# EUROPEAN PATENT SPECIFICATION

(11) **EP 1 905 457 B1**
(45) Date of publication and mention of the grant of the patent: **18.04.2012**
(21) Application number: 07020078.7
(22) Date of filing: 22.12.2000
(51) Int. Cl.: A61K 48/00, A61K 45/00, A61K 38/45, A61K 38/17, A61P 9/10, A61P 7/00

(54) **Pharmaceutical compositions comprising Src and/or Yes and their use**
Pharmazeutische Zusammensetzungen enthaltend Src und / oder Yes und deren Verwendung
Compositions pharmaceutiques comprenant Src et / ou Yes et leur utilisation

(30) Priority: 22.12.1999 US 470881; 29.03.2000 US 538248
(43) Date of publication of application: 02.04.2008
(62) Divisional of application: 00990365.9
(73) Proprietor: THE SCRIPPS RESEARCH INSTITUTE, La Jolla, CA 92037 (US)
(72) Inventor: Cheresh, David, A., Encinitas CA 92024 (US); Eliceiri, Brian, Carlsbad, CA 92009 (US); Paul, Robert, 81371 München (DE)
(74) Representative: Schnappauf, Georg

(56) References cited:
- WO-A-98/50356
- WO-A-98/53051
- WO-A-99/17770
- US-A- 5 922 697
- ELICEIRI B P ET AL: "Selective requirement for Src kinases during VEGF-induced angiogenesis and vascular permeability." MOLECULAR CELL. UNITED STATES DEC 1999, vol. 4, no. 6, December 1999 (1999-12), pages 915-924, XP001152671 ISSN: 1097-2765
- WANG Y D ET AL: "Inhibitors of src tyrosine kinase: the preparation and structure-activity relationship of 4-anilino-3-cyanoquinolines and 4-anilinoquinazolines." BIOORGANIC & MEDICINAL CHEMISTRY LETTERS. ENGLAND 6 NOV 2000, vol. 10, no. 21, 6 November 2000 (2000-11-06), pages 2477-2480, XP004224243 ISSN: 0960-894X

## Description

### Cross-reference to Related Applications

This application claims priority to U.S. Patent Application Serial Nos. 09/470,881, filed Dec. 22, 1999, and 09/538;248, filed March 29, 2000, both of which claim priority to International Patent.Application Number PCT/US99/11780, designating the United States of America and filed May 28, 1999, which claims priority to United States Provisional Application for Patent Serial No. 60/087,220 filed May 29, 1998.

### Statement of Government Rights

Some of the work disclosed has been supported in part by grants from the NIH on behalf of The United States of America. Therefore, the government of the United States of America may have certain rights in the invention.

### Technical Field

The present invention relates generally to the field of medicine, and relates specifically to compositions for modulating and inhibiting vascular permeability (VP).

### Background

Angiogenesis is a process of tissue vascularization that involves the growth of new developing blood vessels into a tissue, and is also referred to as neovascularization. The process is mediated by the infiltration of endothelial cells and smooth muscle cells. The process is believed to proceed in any one of three ways: the vessels can sprout from pre-existing vessels, de-novo development of vessels can arise from precursor cells (vasculogenesis), or existing small vessels can enlarge in diameter. Blood et al., Bioch. Biophys. Acta, 1032:89-118 (1990). For angiogenesis to occur, endothelial cells must first degrade and cross the blood vessel basement membrane in a similar manner used by tumor cells during invasion and metastasis formation. Angiogenesis is generally absent in adult or mature tissues, although it does occur in wound healing and in the corpus luteum growth cycle. See, for example, Moses et al., Science, 248:1408-1410 (1990).

While angiogenesis is an important process in neonatal growth, it is also important in wound healing and is a factor in the pathogenesis of a large variety of clinical diseases including tissue inflammation, arthritis, tumor growth, diabetic retinopathy, macular degeneration by neovascularization of the retina, and like conditions. These clinical manifestations associated with angiogenesis are referred to as angiogenic diseases. Folkman et al., Science, 235:442-447 (1987).

It has been proposed that inhibition of angiogenesis would be a useful therapy for restricting tumor growth. Inhibition of angiogenesis has been proposed by (1) inhibition of release of "angiogenic molecules" such as bFGF (basic fibroblast growth factor), (2) neutralization of angiogenic molecules, such as by use of anti-βbFGF antibodies, (3) use of inhibitors of vitronectin receptor αᵥβ₃, and (4) inhibition of endothelial cell response to angiogenic stimuli. This latter strategy has received attention, Folkman et al., Cancer Biology, 3:89-96 (1992), have described several endothelial cell response inhibitors, including collagenase inhibitor, basement membrane turnover inhibitors, angiostatic steroids, fungal-derived angiogenesis inhibitors, platelet factor 4, thrombospondin, arthritis drugs such as D-penicillamine and gold thiomalate, vitamin D₃ analogs, alpha-interferon, and the like that might be used to inhibit angiogenesis. For additional proposed inhibitors of angiogenesis, see Blood et al., Bioch. Biophys. Acta., 1032:89-118 (1990), Moses et al., Science, 248:1408-1410 (1990), Ingber et al., Lab. Invest., 59:44-51 (1988), and United States Patent No. 5,092,885, No. 5,112,946, No. 5,192,744, No. 5,202,352, No. 5,753,230 and No. 5,766,591. None of the inhibitors of angiogenesis described in the foregoing references involve the Src proteins, however.

It has been previously reported that angiogenesis depends on the interaction between vascular integrins and extracellular matrix proteins. Brooks et al., Science, 264:569-571 (1994). Furthermore, it was reported that programmed cell death (apoptosis) of angiogenic vascular cells is initiated by the interaction, which would be inhibited by certain antagonists of the vascular integrin αᵥβ₃. Brooks et al., Cell, 79:1157-1164 (1994). More recently, it has been reported that the binding of matrix metalloproteinase-2 (MMP-2) to vitronectin receptor (αᵥβ₅) can be inhibited using αᵥβ₅ antagonists, and thereby inhibit the enzymatic function of the proteinase. Brooks et al., Cell, 85:683-693 (1996). The αᵥ integrins have been identified as important components in endothelial cell survival in angiogenic blood vessels. Specific integrin αᵥ integrin antagonists block discrete growth-factor induced angiogenesis pathways. For example, vascular endothelial growth factor (VEGF)-induced angiogenesis is blocked by integrin αᵥβ₅ antagonists, while basic fibroblast growth factor (bFGF)-induced angiogenesis is blocked by integrin αᵥβ₃ antagonists.

The brain vasculature is characterized by a highly restrictive blood-brain barrier that prohibits small molecules from extravasating into the surrounding brain tissue. The nature of the blood-brain barrier in mammals has been of special concern with pharmacological studies, as many drugs are routinely prevented from passing from the vasculature to the brain tissues because of the highly restrictive blood-brain barrier. The present invention involves the unexpected discovery that VP, as measured by vascular leakage of blood, can be modulated by Src or Yes. Moreover, VP has been associated with angiogenesis and other pathologies. Inflammation induced increased vascular permeability is associated with edema and swelling.

A requirement for Src tyrosine kinase activity for VEGF- but not bFGF- induced angiogenesis demonstrated that significant differences in regulation and activation signals between these pathways exist, in both chick embryo and mouse models. Eliceiri et al., Molecular Cell, 4: 915-924 (1999).

Changes in vascular permeability due to angiogenic signals from tumor cells have provided a model for examining the signal pathways related to cancer, however, vascular permeability due to injury, disease or other trauma to the blood vessels is a major cause of vascular leakage and edema associated with tissue damage. For example, cerebrovascular disease associated with cerebrovascular accident (CVA) or other vascular injury in the brain or spinal tissues are the most common cause of neurologic disorder, and a major source of disability. Typically, damage to the brain or spinal tissue in the region of a CVA involves vascular leakage and/or edema. Typically, CVA can include injury caused by brain ischemia, interruption of normal blood flow to the brain; cerebral insufficiency due to transient disturbances in blood flow; infarction, due to embolism or thrombosis of the intra- or extracranial arteries; hemorrhage; and arteriovenous malformations. Ischemic stroke and cerebral hemorrhage can develop abruptly, and the impact of the incident generally reflects the area of the brain damaged. (See The Merck Manual, 16th ed. Chp. 123, 1992).

Other than CVA, central nervous system (CNS) infections or disease can also effect the blood vessels of the brain and spinal column, and can involve inflammation and edema, as in for example bacterial meningitis, viral encephalitis, and brain abscess formation. (See The Merck Manual, 16th ed. Chp. 125, 1992). Systemic disease conditions can also weaken blood vessels and lead to vessel leakage and edema, such as diabetes, kidney disease, atherosclerosis, and the like. Thus, vascular leakage and edema are critical pathologies, distinct from and independent of cancer, which are in need of effective specific therapeutic intervention in association with a variety of injury, trauma or disease conditions.

We have discovered that selective inhibition of Src family tyrosine kinase activity reduces injury or trauma associated VP increase in tissues, and results in amelioration of pathology related to blood vessel leakage and/or edema.

### Summary of the Invention

The present invention is directed to modulation of vascular permeability (VP) by tyrosine kinase Src, also referred to generically herein as Src, or the tyrosine kinase Yes, also referred to generically herein as Yes, or by the selective inhibition of Src family tyrosine kinase activity.

Thus, one aspect of the invention is a pharmaceutical composition comprising tyrosine kinase proteins Src and Yes together with a pharmaceutically acceptable carrier.

In compositions which comprise active Src and Yes kinase proteins, the expected modulation is a potentiation or increase in vascular permeability of the blood vessels in a target tissue. Where the desired Src protein is an active kinase, a preferred Src is Src-A. Another preferred active Src protein is one in which the amino acid residue at position 527 of the Src protein is any amino acid residue except for tyrosine, serine or threonine. The preferred active Yes protein will have the kinase activity of wild-type human Yes, such as that or the Yes-1 protein. Another preferred active Yes is one in which the kinase inactivating phosphorylation site of the Yes protein is mutated to abolish or minimize inactivating phosphorylation, similar to a mutation of amino acid residue 527 of Src to any amino acid residue except for tyrosine, serine or threonine.

Where the composition comprises Src and Yes protein that are inactive kinase proteins, the expected modulation is an inhibition or decrease in vascular permeability of the blood vessels in the target tissue. When the desired Src protein is an inactive protein, a preferred Src is Src 251. A further preferred inactive Src is Src K295M. A preferred inactive Yes protein will have diminished kinase activity as compared with the wild-type protein.

A further aspect of the claimed invention is a pharmaceutical composition comprising an inactive tyrosine kinase protein Yes or a mixture of inactive tyrosine kinase proteins Src and Yes for use in a method for modulating vascular permeability in a tissue suffering from a disease condition and comprising contacting said tissue with a therapeutically effective VP modulating amount of said pharmaceutical composition, wherein the disease condition is selected from the group consisting of stroke induced damages, growth of tumors, myocardial infarction, arteriosclerosis, brain edema, cerebrovascular disease or trauma, rheumatoid arthritis, diabetic retinopathy, arthritis, inflammatory diseases, chronic articular rheumatism and psoriasis, restenosis, cerebral hemorrhage, brain and spinal trauma, hypoxia-induced brain and spinal injury, inflammatory disorders of the CNS, viral or bacterial infections of the CNS, autoimmune disorders, diseases with a chronic increase in blood brain barrier permeability, adult respiratory distress syndrome (ARDS), and metastasis formation.

A further aspect of the claimed invention is a pharmaceutical composition comprising an active tyrosine kinase protein Yes or a mixture of active tyrosine kinase proteins Src and Yes for use in a method for modulating vascular permeability in a tissue suffering from a disease condition and comprising contacting said tissue with a therapeutically effective VP modulating amount of said pharmaceutical composition, wherein the disease condition is selected from the group consisting of ischemic limbs and chronic wounds.

A further aspect of the claimed invention is a pharmaceutical composition comprising a Src family tyrosine kinase inhibitor for use in a method for ameliorating tissue damage related to vascular leakage or edema comprising contacting said tissue with a vascular permeability modulating amount of said pharmaceutical composition, wherein the Src family tyrosine kinase inhibitor is selected from the group consisting of an inactive Yes protein, a mixture of an inactive Src protein and an inactive Yes protein, and a mixture of an inactive Yes protein and/or an inactive Src protein together with a chemical inhibitor selected from PP1, PP2, PD173955, AGL1872, PD162531, Radicicol R2146, and Geldanamycin.

A further aspect of the claimed invention is an article of manufacture comprising packaging material and a pharmaceutical composition contained within said packaging material, wherein said pharmaceutical composition is capable of modulating vascular permeability in a tissue suffering from a disease condition, wherein said packaging material comprises a label which indicates that said pharmaceutical composition can be used for treating disease conditions by modulating vascular permeability, and wherein said pharmaceutical composition comprises a tyrosine kinase Src protein and Yes protein, in a pharmaceutically acceptable carrier.

A further aspect of the claimed invention is an article of manufacture comprising packaging material and a pharmaceutical composition contained within said packaging material, wherein said pharmaceutical composition is capable of modulating vascular permeability in a tissue suffering from a disease condition, wherein said packaging material comprises a label which indicates that said pharmaceutical composition can be used for treating disease conditions by modulating vascular permeability, and wherein said pharmaceutical composition comprises a tyrosine kinase Yes protein, in a pharmaceutically acceptable carrier.

A further aspect of the claimed invention is an article of manufacture comprising packaging material and a pharmaceutical composition contained within said packaging material, wherein said pharmaceutical composition is capable of modulating vascular permeability increase in a tissue suffering from a disease condition, wherein said packaging material comprises a label which indicates that said pharmaceutical composition can be used for treatment of vascular leakage or edema associated disease conditions, and wherein said pharmaceutical composition comprises a Src family tyrosine kinase inhibitor and a pharmaceutically acceptable carrier therefore, wherein the Src family tyrosine kinase inhibitor is selected from the group consisting of an inactive Yes protein, a mixture of an inactive Src protein and an inactive Yes protein, and a mixture of an inactive Yes protein and/or an inactive Src protein together with a chemical inhibitor selected from PP1, PP2, PD173955, AGL1872, PD162531, Radicicol R2146, and Geldanamycin.

It is envisioned that a mixture of Src and Yes protein can combine active and inactive forms of protein, depending upon the level of modulation desired, and the coordinated effect on angiogenesis and VP desired, according to the teaching of the present invention.

A composition providing the Src or Yes protein can contain purified protein, biologically active fragments of natural protein, recombinantly produced Src or Yes protein or protein fragments or fusion proteins, or mixtures thereof.

Where the Src or Yes protein is inactivated or inhibited, the modulation is an inhibition of VP. Where the Src or Yes protein is active or activated, the modulation is a potentiation of VP.

Where the therapeutically effective VP modulating effect desired is an increase or potentiation of VP, it is contemplated that active forms of Src protein and/or Yes protein can be administered.

The tissue to be treated can be any tissue in which modulation of VP is desirable. Therapeutic treatment is accomplished by contacting the target tissue with an effective amount of the desired modulating composition, and allowed sufficient time of contact for the protein components of the pharmaceutical to enter the target tissue. For VP inhibition, it is useful to treat diseased tissue where deleterious vascular leaking is occurring. Exemplary tissues include inflamed tissue, tissues associated with stroke, myocardial infarction, or other blockage of normal flow, tissues undergoing restenosis, and the like tissues.

For potentiation, it is useful to treat patients with ischemic limbs in which there is poor circulation in the limbs from diabetic or other conditions, or for potentiating the administration of drugs to the brain across the blood-brain barrier. Patients with chronic wounds which do not heal and therefore could benefit from the increase in vascular cell proliferation and neovascularization as modulated by VP can be treated as well.

Where the article of manufacture is packaged to indicate a potentiating VP modulating effect, Src and Yes are in active form. A preferred active Src is Src-A protein. Another preferred active Src protein is one in which the amino acid residue at position 527 of the Src protein is any amino acid residue except for tyrosine, serine or threonine.

A preferred inactive Src is Src 251 protein. Another preferred inactive Src protein is Src K295M.

Tissue damage related to vascular leakage and/or edema associated with deleterious changes in vascular permeability can be ameliorated by a Src family tyrosine kinase inhibitor. To that end, an effective, vascular permeability modulating amount of a Src family tyrosine kinase inhibitor is administered to a tissue in need of such treatment. Tissue damage due to vascular leakage or edema can be reduced in this manner.

Any pathology which involves deleterious injury-induced increase in vascular permeability and tissue damage due to vascular leakage or edema can be treated according to the invention. Such pathological events can include trauma to the blood vessels such as physical ligation, blockage, separation, occlusion, trauma, and the like. Other systemic pathological events such as atherosclerosis, diabetic retinopathy, inflammatory disease due to infection by microbial agents, arthritis and the like are also appropriately treated by the invention.

The present invention is useful for treating cerebrovascular disease or trauma by ameliorating tissue damage due to increased vascular leakage and/or edema associated therewith. In particular, the present invention is useful for ameliorating tissue damage associated with Vascular Endothelial Growth Factor (VEGF)-induced Src mediated increase in vascular permeability. However, the invention is not limited to VEGF-induced increases in vascular permeability, and is also appropriate for modulating Src family tyrosine kinase mediated increase in vascular permeability in response to other regulatory signals.

In particular, by inhibiting tyrosine kinase Src, (also referred to generically herein as Src), and the closely related tyrosine kinase Yes, (also referred to generically herein as Yes) treated tissues can be specifically modulated to inhibit therein an increase in vascular permeability associated with injury or disease.

A suitable Src family tyrosine kinase inhibitor for purposes of the present invention is a chemical inhibitor selected from the group consisting of PP1, PP2, PD173955, AGL1872, PD162531, Radicicol R2146, and Geldanamycin. Other chemical inhibitors of Src family tyrosine kinases are also appropriate for use in the invention.

Vascular permeability in tissue can also be modulated by administering to the tissue a Src family tyrosine kinase inhibitor that is a protein inhibitor, such as an inactive Src protein like Src K295M or Src 251, or an inactive Yes protein, or an active c-terminal Src Kinase (CSK) protein.

An article of manufacture of the invention may contain as part of the pharmaceutical composition a Src family tyrosine kinase inhibitor that is a chemical inhibitor. In particular, a preferred chemical Src family tyrosine kinase inhibitor is selected from the group consisting of PP1, PP2, PD173955, AGL1872, PD162531, Radicicol R2146, and Geldanamycin, or compounds with similar Src inhibiting activity. A most preferred inhibitor is PP1.

Articles of manufacture may include one or more pharmaceutical compositions that contain therapeutic Src family tyrosine kinase inhibitors, or sub-therapeutic amounts of more than one Src family tyrosine kinase inhibitors, in a pharmaceutically acceptable carrier.

Pharmaceutical compositions of the articles of manufacture of the invention may comprise mixtures of one or more sub-therapeutically effective VP modulating amount of a Src family tyrosine kinase inhibitor, which act together to provide a VP reducing effect on treated tissue. The pharmaceutical composition of the article of manufacture can vary depending upon the desired modulatory effect, and the packaging labeling will correspondingly vary as well.

The pharmaceutical composition of the article of manufacture can vary depending upon the desired modulatory or inhibiting effect, and the packaging labeling will correspondingly vary as well.

### Brief Description of the Drawings

In the drawings forming a portion of this disclosure:

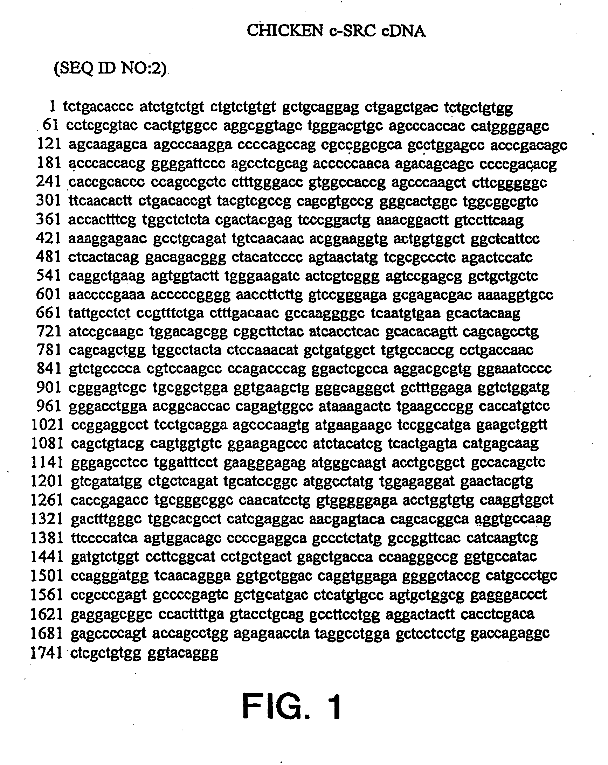

FIG. 1 is a cDNA sequence of chicken c-Src which is the complete coding sequence with the introns deleted as first described by Takeya et al., Cell, 32:881-890 (1983). The sequence is accessible through GenBank Accession Number J00844. The sequence contains 1759 nucleotides with the protein coding portion beginning and ending at the respective nucleotide positions 112 and 1713 (SEQ ID NO:2).

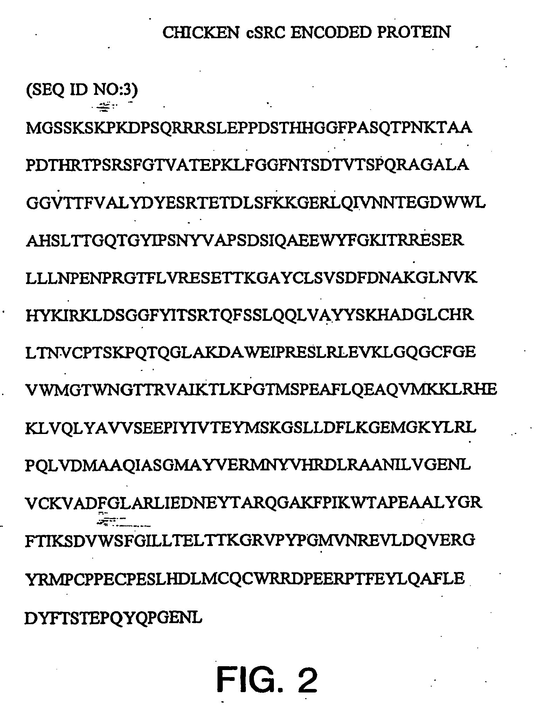

FIG. 2 is the encoded amino acid residue sequence of chicken c-Src of the coding sequence shown in FIG. 1 (SEQ ID NO:3).

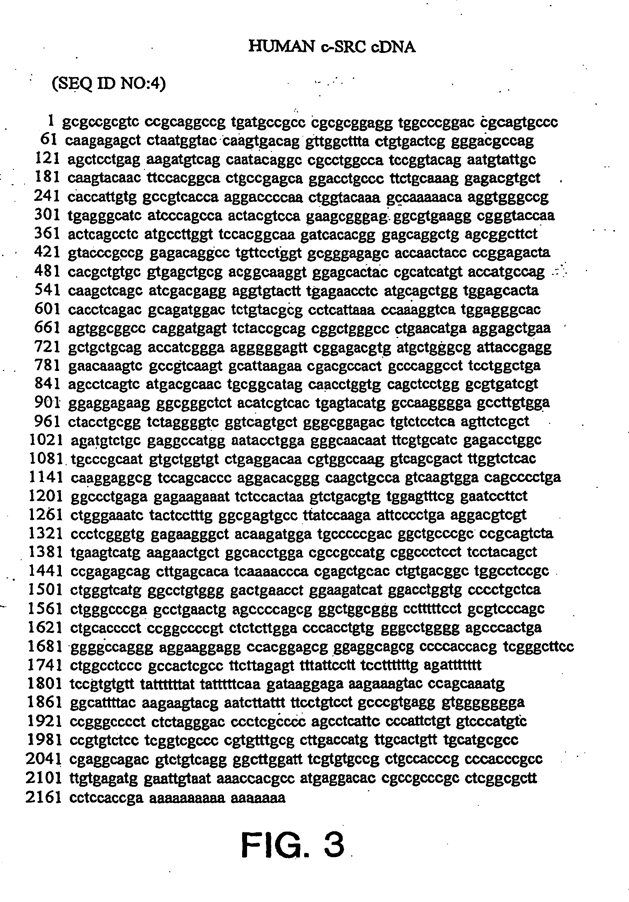

FIG. 3 is a cDNA sequence of human c-Src which as first described by Braeuninger et al., Proc. Natl. Acad. Sci., USA, 88:10411-10415 (1991). The sequence is accessible through GenBank Accession Number X59932 X71157. The sequence contains 2187 nucleotides with the protein coding portion beginning and ending at the respective nucleotide positions 134 and 1486 (SEQ ID NO:4).

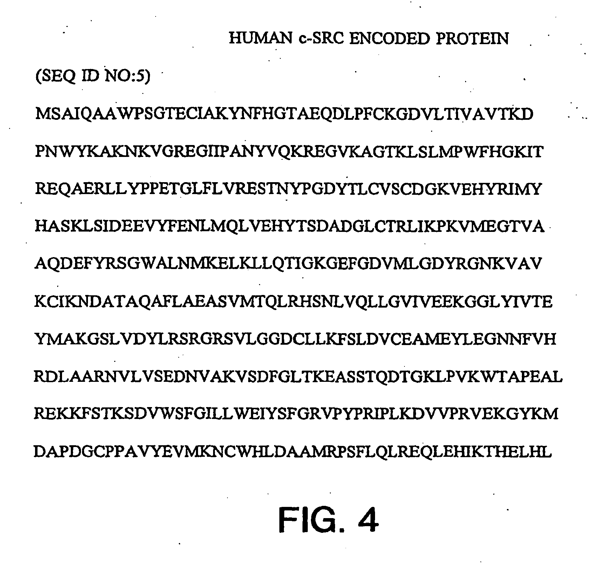

FIG. 4 is the encoded amino acid residue sequence of human c-Src of the coding sequence shown in FIG. 3 (SEQ ID NO:5).

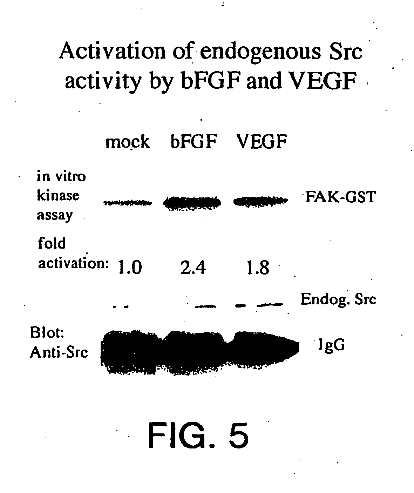

FIG. 5 illustrates the activation of endogenous Src by bFGF or VEGF as described in Example 4. The top portion of the figure indicates the results of an in vitro kinase assay with the fold activation of endogenous c-Src by either bFGF and VEGF. The bottom of the figure is the kinase assay blot probed with an anti-Src antibody as a loading control for equivalent Src and IgG content.

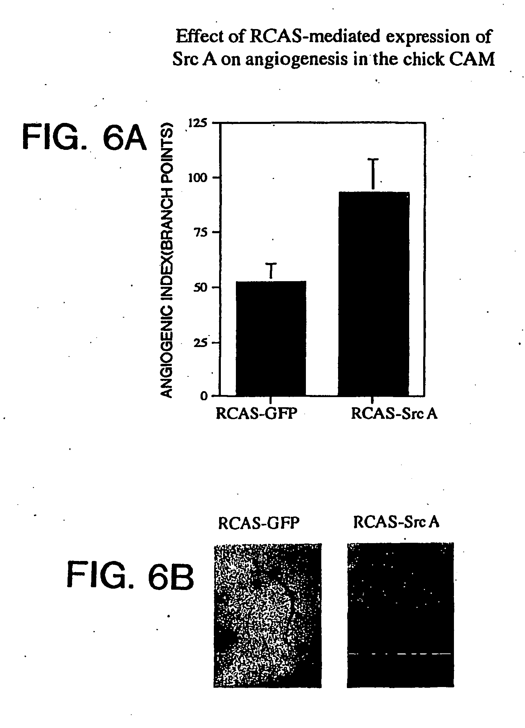

FIG. 6 illustrates the effect of retrovirus-mediated gene expression of c-Src A on angiogenesis in the Chick CAM as described in Example 4. Nine-day-old chick CAMs were exposed to RCAS-Src A (active mutated c-Src) or control RCAS-GFP (Green Fluorescent Protein; a fluorescent indicator protein) retroviruses or buffer for 72 h. The level of angiogenesis was quantified as shown in FIG. 6A with representative photomicrographs (4x) in FIG. 6B corresponding to each treatment taken with a stereomicroscope.

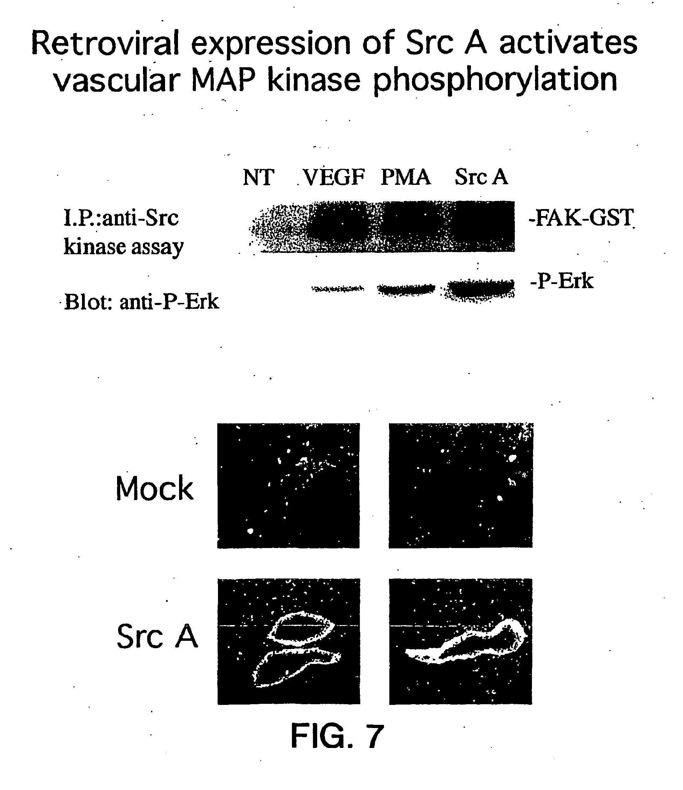

FIG. 7 illustrates the retroviral expression of c-Src A in activating vascular MAP kinase phosphorylation. FIG. 7A shows tissue extracts of 10 day-old chick CAMs that had been exposed to VEGF or PMA for 30 minutes or infected with c-src A retrovirus for 48 hours. NT stands for no treatment. Src was immunoprecipitated from equivalent amounts of total protein extract and subjected to an in vitro immune complex kinase assay using a FAK-GST fusion protein as a substrate, electrophoresed and transferred to nitrocellulose. Aliquots of the above whole tissue lysates were also measured for endogenous ERK phosphorylation by immunoblotting with an anti-phospho-ERK antibody. FIG. 7B shows 10 day old CAMs that were infected with either mock RCAS or RCAS containing SRC A. After two days, CAMs were dissected, cryopreserved in OCT and sectioned at 4 µm. Sections were immunostained with an anti-phosphorylated ERK antibody (New England Biolabs), washed and detected with a goat anti-rabbit FITC-conjugated secondary antibody. Florescent images were captured on a cooled-CCD camera (Princeton Inst.)

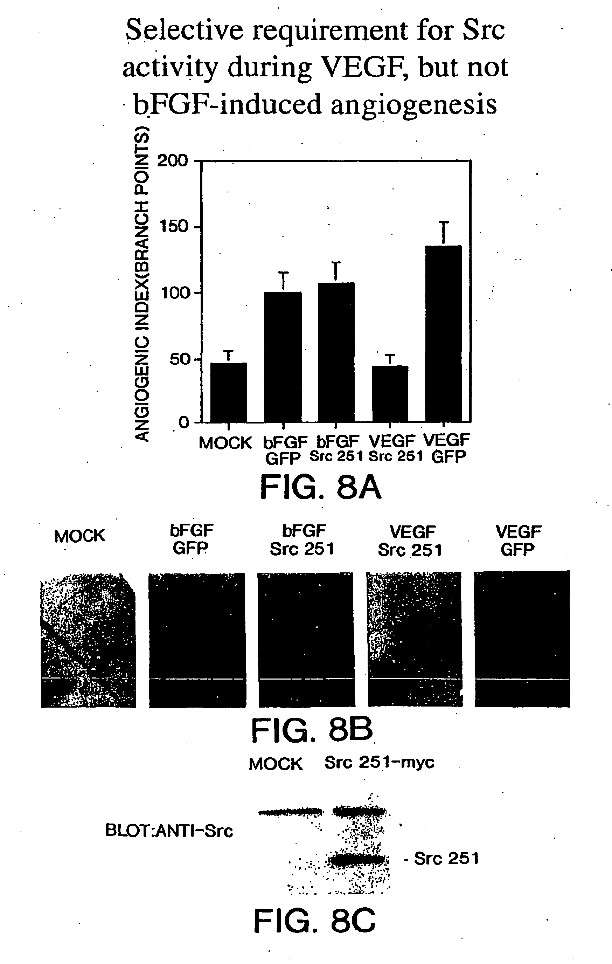

FIG. 8 illustrates the selective requirement for Src activity during VEGF, but not bFGF-induced angiogenesis. Nine day old chick CAMs were exposed to RCAS-Src 251 or control RCAS-GFP retroviruses or buffer for 20 hours and then incubated for an additional 72 hours in the presence or absence of bFGF or VEGF. The level of angiogenesis was quantified (FIG. 8A) as described above, and representative photomicrographs (6x) were taken with a stereomicroscope as shown in FIG. 8B. FIG. 8C shows a blot probed with an anti-Src antibody to confirm the expression of Src 251 in transfected cells as compared to mock treatments.

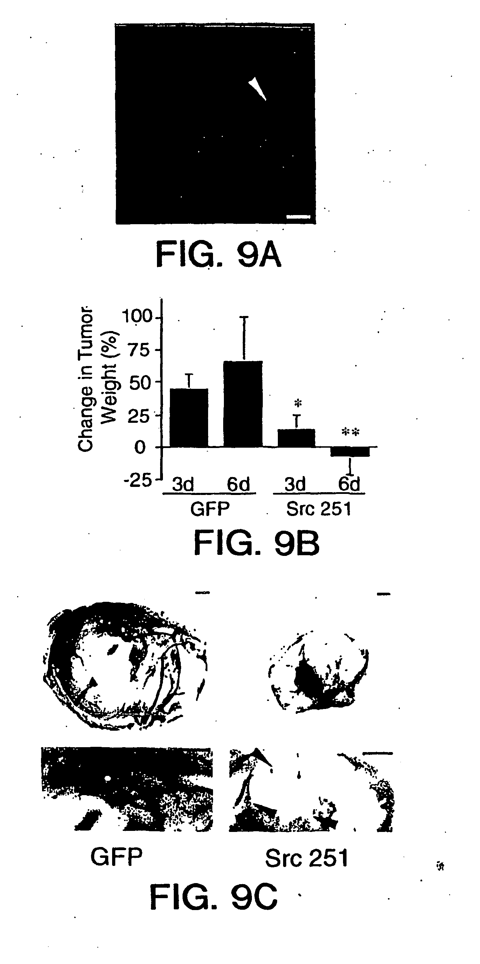

FIG. 9 illustrates the results of retroviral delivery of RCAS-Src 251 to human tumors. FIG. 9A is a micrograph that shows human medulloblastoma tumor fragment infected with RCAS-GFP (RCAS-Green Fluorescent Protein) expressing GFP exclusively in the tumor blood vessels (arrowhead) as detected by optical sectioning with a Bio Rad laser confocal scanning microscope (bar=500 µm). FIG. 9B depicts data from tumors treated with topical application of retrovirus, which were allowed to grow for 3 or 6 days after which they were resected and wet weights determined. Data are expressed as the mean change in tumor weight (from the 50 mg tumor starting weight) +/- SEM of 2 replicates. FIG. 9C depicts in representative micrographs, medulloblastoma tumors surgically removed from the embryos (bar=350 µm). The lower panels are high magnification views of each tumor showing the vasculature of each tumor in detail (bar=350 µm). The arrowhead indicates blood vessel disruption in RCAS-Src251-treated tumors.

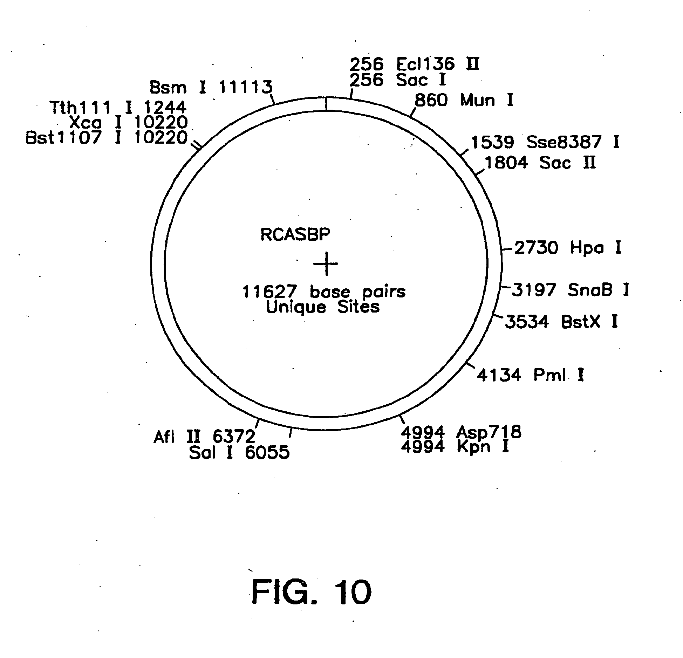

FIG. 10 is a diagram illustrating a restriction map of the RCASBP (RCAS) vector construct (SEQ ID NO:1).

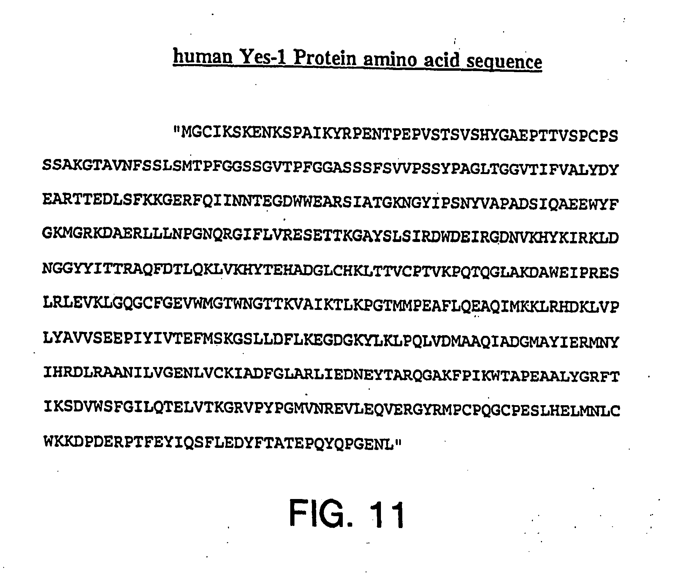

FIG. 11 depicts the encoded amino acid residue sequence of human c-Yes protein in single letter amino acid representation (SEQ ID NO:8).

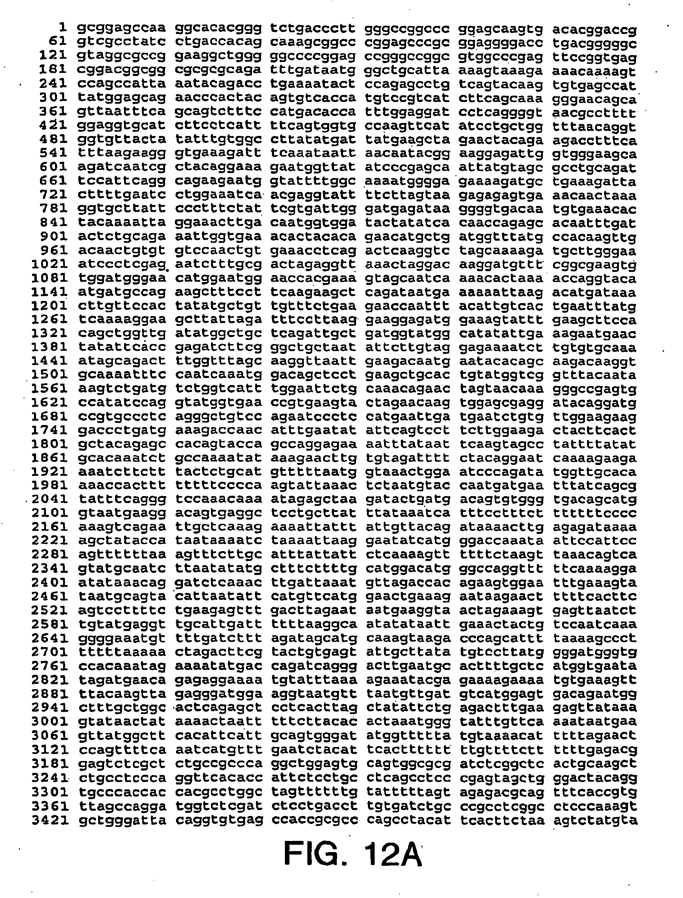

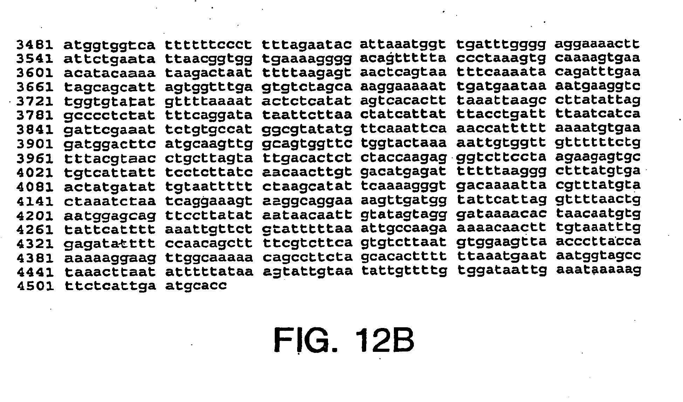

FIG. 12 depicts the nucleic acid sequence of a cDNA encoding for human c-Yes protein. The sequence is accessible through GenBank Accession Number M15990. The sequence contains 4517 nucleotides with the protein coding portion beginning and ending at the respective nucleotide positions 208 and 1839, and translating into to amino acid depicted in FIG. 11 (SEQ ID NO:7).

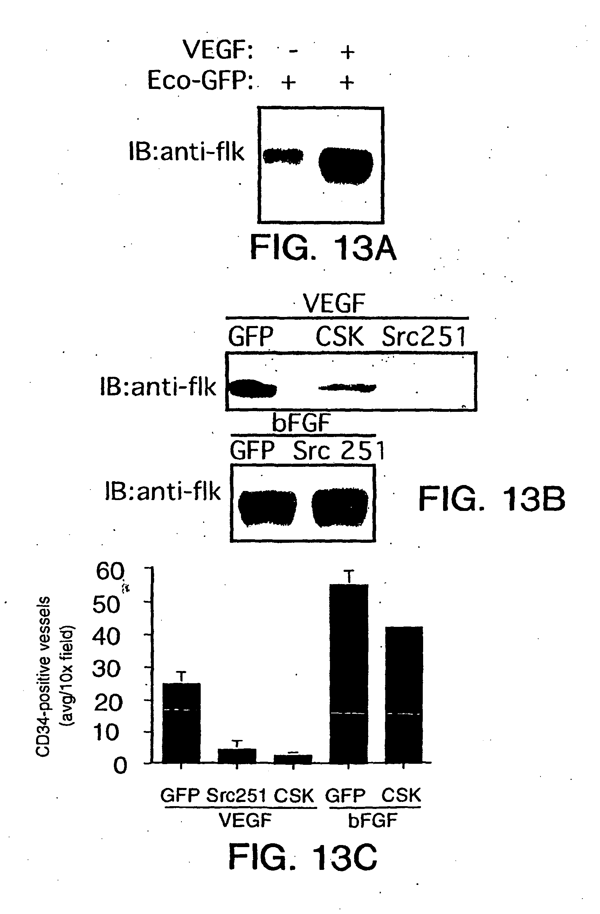

FIG. 13 depicts results from retroviral delivery of Src 251 and CSK in a subcutaneous murine angiogenesis model. FIG. 13A illustrates immunoblotting results for detecting flk expression. FIG. 13B illustrates immunoblotting results from assay for flk under VEGF and bFGF stimulated conditions. FIG. 13C is a graph which plots the number of CD34 positive blood vessels (average of triplicate random fields at 20x) by treatment as stimulated by VEGF and bFGF in the presence of GFP, Src 251, or CSK retrovirus.

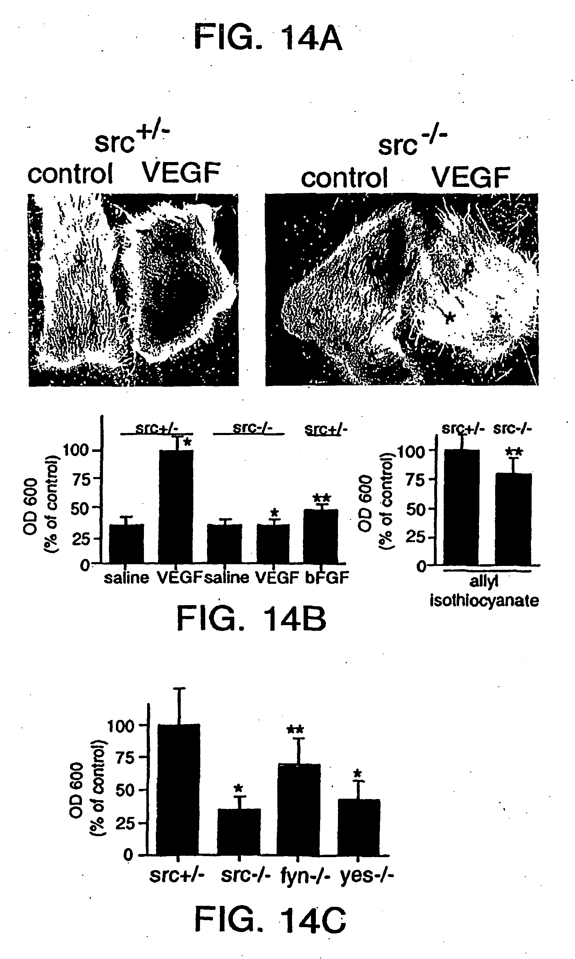

FIG. 14 illustrates results from a modified Miles assay for VP of VEGF in the skin of mice deficient in Src, fyn and Yes. FIG. 14A are photographs of treated ears. FIG. 14B are graphs of experimental results for stimulation of the various deficient mice. FIG. 14C plots the amount of eluted Evan's blue dye by treatment.

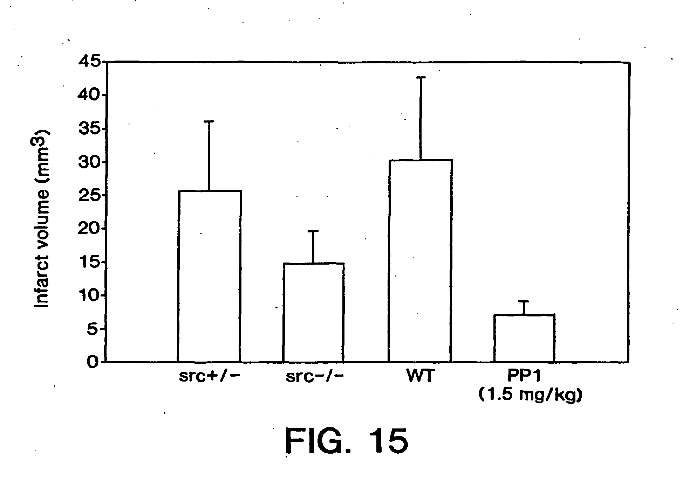

FIG. 15 is a graph depicting the relative size of infarct in Src +/-, Src -/-, wild type (WT), and PP1 treated wild type mice. PP1 treatment consisted of 1.5 mg/kg body weight.

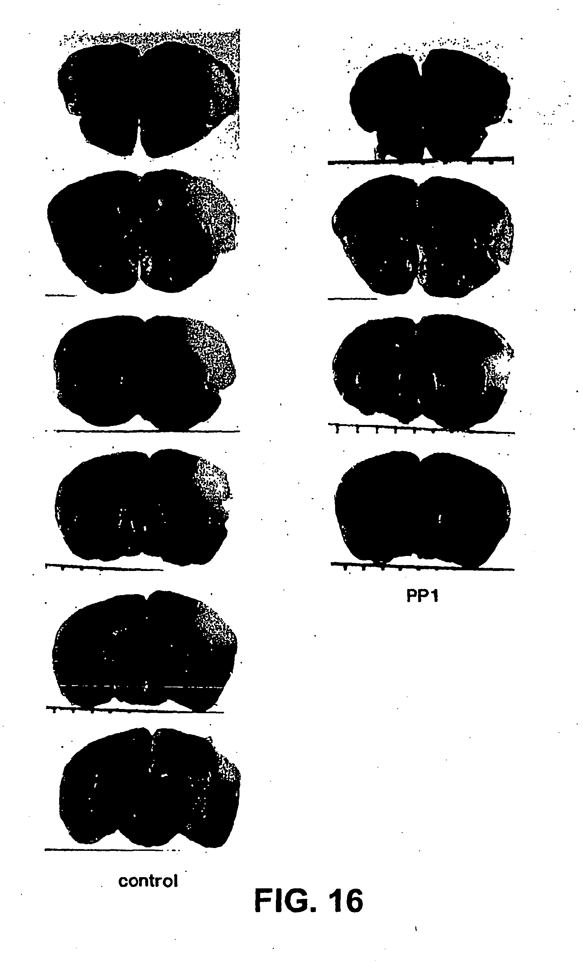

FIG. 16 depicts sequential MRI scans of control and PP1 treated mouse brains showing less brain infarction in PP1 treated animal (right) than in the control animal (left).

### Detailed Description of the Invention

### A. Definitions

Amino Acid Residue: An amino acid formed upon chemical digestion (hydrolysis) of a polypeptide at its peptide linkages. The amino acid residues described herein are preferably in the "L" isomeric form. However, residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxy terminus of a polypeptide. In keeping with standard polypeptide nomenclature (described in J. Biol. chem., 243:3552-59 (1969) and adopted at 37 CFR §1.822(b)(2)).

It should be noted that all amino acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino acid residues.

Polypeptide: refers to a linear series of amino acid residues connected to one another by peptide bonds between the alpha-amino group and carboxy group of contiguous amino acid residues.

Peptide: as used herein refers to a linear series of no more than about 50 amino acid residues connected one to the other as in a polypeptide.

Cyclic peptide: refers to a compound having a heteroatom ring structure that includes several amide bonds as in a typical peptide. The cyclic peptide can be a homodetic "head to tail" cyclized linear polypeptide in which a linear peptide's n-terminus has formed an amide bond with the c-terminal carboxylate of the linear peptide, or it can contain a ring structure in which the polymer is heterodetic and comprises amide bonds and/or other bonds to close the ring, such as disulfide bridges, thioesters, thioamides, guanidino, and the like linkages.

Protein: refers to a linear series of greater than 50 amino acid residues connected one to the other as in a polypeptide.

Fusion protein: refers to a polypeptide containing at least two different polypeptide domains operatively linked by a typical peptide bond ("fused"), where the two domains correspond to peptides not found fused in nature.

Synthetic peptide: refers to a chemically produced chain of amino acid residues linked together by peptide bonds that is free of naturally occurring proteins and fragments thereof.

### B. General Considerations

The present invention relates generally to: (1) the discovery that VEGF induced VP is specifically mediated by the tyrosine kinase proteins Src and Yes, and that VP can be modulated by providing either active or inactive Src or Yes proteins for potentiating or inhibiting angiogenesis, respectively; (2) the further discovery that vascular leakage and/or edema associated with trauma, disease of injury related increase in vascular permeability can be specifically modulated, and ameliorated, by inhibition of Src family tyrosine kinase activity; and (3) the discovery that *in vivo* administration of a Src family tyrosine kinase inhibitor decreases tissue damage due to disease- or injury-related increase in vascular permeability that is not associated with cancer or angiogenesis.

This discovery is important because of the role that vascular permeability plays in a variety of disease processes and in association with angiogenesis, the formation of new blood vessels. Where tissues associated with a disease condition require angiogenesis for tissue growth, it is desirable to inhibit angiogenesis and thereby inhibit the diseased tissue growth. Angiogenesis may be more effectively inhibited by simultaneously inhibiting VP. Where injured tissue requires angiogenesis for tissue growth and healing, it is desirable to potentiate or promote VP and thus angiogenesis, and thereby promote tissue healing and growth.

Where the growth of new blood vessels is the cause of, or contributes to, the pathology associated with a disease tissue, inhibition of VP, and thereby angiogenesis will reduce the deleterious effects of the disease. By inhibiting VP associated with angiogenesis, one can intervene in the disease, ameliorate the symptoms, and in some cases cure the disease.

In certain instances, increased VP is desirable for increasing the efficacy of drug delivery via systemic administration. The blood-brain barrier is a term used to describe the tight regulation of VP, and thus minimal access of even small molecule drugs to the brain from the circulation. The ability to selectively and specifically modulate the permeability of the blood-brain barrier via modulation of the VP of the involved blood vessels will allow the administration of drugs that otherwise would not be able to pass via the circulation into the brain tissues.

Similarly, many stroke induced pathologies and damage are instigated by the sudden increase in VP, and thus the ability to specifically modulate VP will allow for novel and effective treatments to reduce the adverse effects of stroke.

The therapy described herein is effective in part because it is highly selective for VP and not other biological processes.

The present invention relates, in part, to the discovery that angiogenesis is mediated by the tyrosine kinase Src protein, and that angiogenesis can be modulated by providing either active or inactive Src proteins for potentiating or inhibiting angiogenesis, respectively.

This discovery is important because of the role that angiogenesis, the formation of new blood vessels, plays in a variety of disease processes. Where tissues associated with a disease condition require angiogenesis for tissue growth, it is desirable to inhibit angiogenesis and thereby inhibit the diseased tissue growth. Where injured tissue requires angiogenesis for tissue growth and healing, it is desirable to potentiate or promote angiogenesis and thereby promote tissue healing and growth.

Where the growth of new blood vessels is the cause of, or contributes to, the pathology associated with a disease tissue, inhibition of angiogenesis will reduce the deleterious effects of the disease. By inhibiting angiogenesis, one can intervene in the disease, ameliorate the symptoms, and in some cases cure the disease.

Examples of tissue associated with disease and neovascularization that will benefit from inhibitory modulation of angiogenesis include rheumatoid arthritis, diabetic retinopathy, inflammatory diseases, restenosis, and the like. Where the growth of new blood vessels is required to support growth of a deleterious tissue, inhibition of angiogenesis will reduce the blood supply to the tissue and thereby contribute to reduction in tissue mass based on blood supply requirements. Examples include growth of tumors where neovascularization is a continual requirement in order that the tumor grow beyond a few millimeters in thickness, and for the establishment of solid tumor metastases.

Where the growth of new blood vessels is believed to contribute to healing of tissue, potentiation of angiogenesis will assist in healing. Examples include treatment of patients with ischemic limbs in which there is poor circulation in the limbs from diabetes or other conditions. Also contemplated are patients with chronic wounds which do not heal and therefore could benefit from the increase in vascular cell proliferation and neovascularization.

The therapy described herein is effective in part because it is highly selective for angiogenesis and not other biological processes.

As described earlier, angiogenesis includes a variety of processes involving neovascularization of a tissue including "sprouting", vasculogenesis, or vessel enlargement, all of which angiogenesis processes are effected by Src protein. With the exception of traumatic wound healing, corpus luteum formation and embryogenesis, it is believed that the majority of angiogenesis processes are associated with disease processes and therefore the therapy described herein is selective for the disease and does not have deleterious side effects.

The present invention also relates, in part, to the discovery that vascular leakage and/or edema associated with trauma, disease of injury related increase in vascular permeability can be specifically modulated, and ameliorated, by inhibition of Src family tyrosine kinase activity. In particular, the present invention is related to the discovery that the *in vivo* administration of a Src family tyrosine kinase inhibitor decreases tissue damage due to disease- or injury-related increase in vascular permeability that is not associated with cancer or angiogenesis.

While administration of a Src family tyrosine kinase inhibitor modulates VEGF-induced VP increase, the specific inhibition of Src family kinase activity ameliorates damage to surrounding tissues caused by vascular leakage and/or edema, however the Src family kinase signal is activated.

Vascular permeability is implicated in a variety of disease processes independent of any direct association with angiogenesis. For example, many stroke induced pathologies and damage are caused by the sudden increase in VP due to trauma to the blood vessel, and thus the ability to specifically modulate VP will allow for novel and effective treatments to reduce the adverse effects of stroke.

Examples of tissue associated with disease or injury induced vascular leakage and/or edema that will benefit from the specific inhibitory modulation using a Src family kinase inhibitor include rheumatoid arthritis, diabetic retinopathy, inflammatory diseases, restenosis, and the like.

Trauma to the head or spine, and other cerebrovascular accident typically caused by ischemic or hemorrhagic events, are a major cause of neurological disorder and related injury. Brain edema or vascular leakage resulting from such injuries, is a life-threatening pathology which triggers systemic and disseminated damage to the brain and spinal cord (central nervous system; CNS) and the ability to specifically modulate the tissue damaging effects of vascular leakage and edema in such instances is very useful.

CNS infections, meningitis, cerebritis, encephalitis, can all result in the adverse pathology including cerebral edema. Treatment of the underlying infection can be supplemented with specific therapy to reduce vascular leakage or edema.

It has been reported that systemic neutralization of VEGF protein using a VEGF receptor IgG fusion protein reduces infarct size following cerebral ischemia, this effect was attributed to the reduction of VEGF-mediated vascular permeability. N. van Bruggen et al., J. Clin. Inves. 104:1613-1620 (1999). However, VEGF is not the critical mediator of vascular permeability increase that Src has now been discovered to be.

Other diseases or conditions where Src mediated increase in vascular permeability is involved and are thus suitable targets for treatment by the compositions of the present invention may include: cerebral hemorrhage, brain and spinal trauma, hypoxia-induced brain and spinal injury; inflammatory disorders of the CNS: viral or bacterial infections (e.g. meningitis, HIV encephalopathy), autoimmune disorders (e.g. multiple sclerosis); diseases with a chronic increase in blood brain barrier permeability (e.g. Morbus Alzheimer); in surgeries where a temporary impairment of perfusion or oxygenation of tissue is needed, as a protective agent; adult respiratory distress syndrome (ARDS); rheumatoid arthritis; and diabetic retinopathy.

### C. Src Family Tyrosine Kinase Proteins

A tyrosine kinase protein for use in the present invention can vary depending upon the intended use. The terms "Src protein" or "Src" are used to refer collectively to the various forms of tyrosine kinase Src protein described herein, either in active or inactive forms. The terms "Yes protein" or "Yes" are used to refer collectively to the various forms of tyrosine kinase Yes protein described herein, either in active or inactive forms. Also, in the context of the description, reference is also made to Src or Yes encoding nucleic acid genetic sequence or genes. The term "Src family" refers to the group of tyrosine kinases which are related in function and amino acid sequence to Src.

An "active Src protein" refers to any of a variety of forms of Src protein which potentiate angiogenesis or VP. An "active Yes protein" refers to any of a variety of forms of Yes protein which potentiate VP. Assays to measure potentiation of angiogenesis or VP are described herein, and are not to be construed as limiting. A protein is considered active if the level of angiogenesis or VP is at least 10% greater, preferably 25% greater, and more preferably 50% greater than a control level where no protein is added to the assay system.

The preferred assay for measuring potentiation of angiogenesis is the CAM assay using RCAS viral vector as described in the Examples in which the angiogenic index is calculated by counting branch points.

A preferred assay for measuring potentiation of VP is the Miles assay using Evan's blue dye in mice as described in the Examples, in which VP is measured by the amount of Evan's blue dye leaked from blood vessels.

A preferred active Src or Yes protein exhibits tyrosine kinase activity as well. Exemplary active Src or Yes proteins are described in the Examples, and include Src-A and Yes-1.

An "inactive Src protein" refers to any of a variety of forms of Src protein which inhibit angiogenesis or VP. An "inactive Yes protein" refers to any of a variety of forms of Yes protein which inhibit VP. Assays to measure inhibition of VP increase are described herein, and are not to be construed as limiting. A Src protein is considered inactive if the level of angiogenesis is at least 10% lower, preferably 25% lower, and more preferably 50% lower than a control level where no exogenous Src is added to the assay system.

A Src or Yes protein is considered inactive if the level of VP is at least the same as, or 10% lower, preferably 25% lower, and more preferably 50% lower than a control level where no exogenous Src or Yes is added to the assay system.

The preferred assay for measuring inhibition of angiogenesis is the CAM assay using RCAS viral vector as described in the Examples in which the angiogenic index is calculated by counting branch points.

A preferred assay for measuring inhibition of VP is the Miles assay using Evan's blue dye in mice as described in the Examples, in which VP is measured by the amount of Evan's blue dye leaked from blood vessels.

A preferred inactive Src or Yes protein exhibits reduced tyrosine kinase activity as well. Exemplary inactive Src proteins are described in the Examples, and include Src-251 and Src K295M.

A Src protein useful in the present invention can be produced in any of a variety of methods including isolation from natural sources including tissue, production by recombinant DNA expression and purification, and the like.

A gene encoding a Src or Yes protein can be prepared by a variety of methods known in the art, and the invention is not to be construed as limiting in this regard. For example, the natural history of Src is well known to include a variety of homologs from mammalian, avian, viral and the like species, and the gene can readily be cloned using cDNA cloning methods from any tissue expressing the protein. A preferred Src for use in the invention is a cellular protein, such as the mammalian or avian homologs designated c-src. Particularly preferred is human c-src. A preferred Yes for use in the invention is a human cellular protein, c-yes. Particularly preferred is human c-yes-1 encoding for the amino acid sequence as depicted in FIG. 11. The protein Yes-1 of FIG. 11 is encoded for by a segment of the nucleic acid sequence depicted in FIG. 12, and identified as the coding domain segment.

### D. Recombinant DNA Molecules and Expression Systems for Expression of Src or Yes Protein

Several nucleotide sequences are described herein which can be used for expressing Src or Yes protein. These sequences include sequences which encode a Src protein useful in the invention, and various DNA segments, recombinant DNA (rDNA) molecules and vectors constructed for expression of Src protein. These sequences also include sequences which encode a Yes protein useful in the invention, and various DNA segments, recombinant DNA (rDNA) molecules and vectors constructed for expression of Yes protein.

DNA molecules (segments) therefore can comprise sequences which encode whole structural genes, fragments of structural genes, or combination of genes, and transcription units as described further herein.

A preferred DNA segment is a nucleotide sequence which encodes a Src or Yes protein, or both as defined herein, or biologically active fragment thereof.

The amino acid residue sequence and nucleotide sequence of a preferred Src and Yes is described in the Examples.

A preferred DNA segment codes for an amino acid residue sequence substantially the same as, and preferably consisting essentially of, an amino acid residue sequence or portions thereof corresponding to a Src or Yes protein described herein. Representative and preferred DNA segments are further described in the Examples.

The amino acid residue sequence of a protein or polypeptide is directly related via the genetic code to the deoxyribonucleic acid (DNA) sequence of the structural gene that codes for the protein. Thus, a structural gene or DNA segment can be defined in terms of the amino acid residue sequence, i.e., protein or polypeptide, for which it codes.

An important and well known feature of the genetic code is its redundancy. That is, for most of the amino acids used to make proteins, more than one coding nucleotide triplet (codon) can code for or designate a particular amino acid residue. Therefore, a number of different nucleotide sequences may code for a particular amino acid residue sequence. Such nucleotide sequences are considered functionally equivalent since they can result in the production of the same amino acid residue sequence in all organisms. Occasionally, a methylated variant of a purine or pyrimidine may be incorporated into a given nucleotide sequence. However, such methylation do not affect the coding relationship in any way.

A nucleic acid is any polynucleotide or nucleic acid fragment, whether it be a polyribonucleotide of polydeoxyribonucleotide, i.e., RNA or DNA, or analogs thereof. In preferred embodiments, a nucleic acid molecule is in the form of a segment of duplex DNA, i.e, a DNA segment, although for certain molecular biological methodologies, single-stranded DNA or RNA is preferred.

DNA segments are produced by a number of means including chemical synthesis methods and recombinant approaches, preferably by cloning or by polymerase chain reaction (PCR). DNA segments that encode portions of a Src protein can easily be synthesized by chemical techniques, for example, the phosphotriester method of Matteucci et al., J. Am. Chem. Soc., 103:3185-3191, 1981, or using automated synthesis methods. In addition, larger DNA segments can readily be prepared by well known methods, such as synthesis of a group of oligonucleotides that define the DNA segment, followed by hybridization and ligation of oligonucleotides to build the complete segment. Alternative methods include isolation of a preferred DNA segment by PCR with a pair of oligonucleotide primers used on a cDNA library believed to contain members which encode a Src protein.

Of course, through chemical synthesis, any desired modifications can be made simply by substituting the appropriate bases for those encoding the native amino acid residue sequence. This method is well known, and can be readily applied to the production of the various different "modified" Src proteins described herein.

Furthermore, DNA segments consisting essentially of structural genes encoding a Src or Yes protein can be subsequently modified, as by site-directed or random mutagenesis, to introduce any desired substitutions.

### 1. Cloning a Src or Yes Gene

A Src or Yes gene can be cloned from a suitable source of genomic DNA or messenger RNA (mRNA) by a variety of biochemical methods. Cloning these genes can be conducted according to the general methods described in the Examples and as known in the art.

Sources of nucleic acids for cloning a Src or Yes gene can include genomic DNA or messenger RNA (mRNA) in the form of a cDNA library, from a tissue believed to express these proteins. A preferred tissue is human lung tissue, although any other suitable tissue may be used.

A preferred cloning method involves the preparation of a cDNA library using standard methods, and isolating the Src-encoding, or Yes-encoding nucleotide sequence by PCR amplification using paired oligonucleotide primers based on the nucleotide sequences described herein. Alternatively, the desired cDNA clones can be identified and isolated from a cDNA or genomic library by conventional nucleic acid hybridization methods using a hybridization probe based on the nucleic acid sequences described herein. Other methods of isolating and cloning suitable Src or Yes encoding nucleic acids are readily apparent to one skilled in the art.

### 2. Gene Transfer and/or Expression Vectors

A recombinant DNA molecule (rDNA) containing a DNA segment encoding a Src or Yes protein, or both, is described herein. An expressible rDNA can be produced by operatively (in frame, expressible) linking a vector to a Src or Yes encoding DNA segment. Thus, a recombinant DNA molecule is a hybrid DNA molecule comprising at least two nucleic acids of a nucleotide sequences not normally found together in nature.

The choice of vector to which a DNA segment is operatively linked depends directly, as is well known in the art, on the functional properties desired, e.g., protein expression, and the host cell to be transformed. Typical considerations in the art of constructing recombinant DNA molecules. A vector is at least capable of directing the replication, and preferably also expression, of a structural gene included in the vector DNA segments, to which it is operatively linked.

Where an expression vector contains both an expressible src and yes nucleic acid sequence, both genes may be regulated by the same regulatory elements upstream of the first gene, or each individually regulated by separate regulatory elements.

Both prokaryotic and eukaryotic expression vectors are familiar to one of ordinary skill in the art of vector construction, and are described by Ausebel, et al., in Current Protocols in Molecular Biology, Wiley and Sons, New York (1993) and by Sambrook et al., Molecular Cloning: A Laboratory Manual, Cold Spring Harbor Laboratory, (1989). These references also describe many of the general recombinant DNA methods referred to herein.

In one embodiment, a vector includes a procaryotic replicon, i.e., a DNA sequence having the ability to direct autonomous replication and maintenance of the recombinant DNA molecule extrachromosomally in a procaryotic host cell, such as a bacterial host cell, transformed therewith. Such replicons are well known in the art. In addition, those embodiments that include a procaryotic replicon also include a gene whose expression confers drug resistance to a bacterial host transformed therewith. Typical bacterial drug resistance genes are those that confer resistance to ampicillin or tetracycline.

Those vectors that include a procaryotic replicon can also include a procaryotic promoter capable of directing the expression (transcription and translation) of a structural gene in a bacterial host cell, such as E. coli, transformed therewith. A promoter is an expression control element formed by a DNA sequence that permits binding of RNA polymerase and transcription to occur. Promoter sequences compatible with bacterial hosts are typically provided in plasmid vectors containing convenient restriction sites for insertion of a DNA segment. Typical of such vector plasmids are pUC8, pUC9, pBR322 and pBR329 available from Biorad Laboratories, (Richmond, CA), pRSET available from Invitrogen (San Diego, CA) and pPL and pKK223 available from Pharmacia, Piscataway, N.J.

Expression vectors compatible with eukaryotic cells, preferably those compatible with vertebrate cells, can also be used to form the recombinant DNA molecules. Eukaryotic cell expression vectors are well known in the art and are available from several commercial sources. Typically, such vectors are provided containing convenient restriction sites for insertion of the desired DNA segment. Typical of such vectors are pSVL and pKSV-10 (Pharmacia), pBPV-1/pML2d (International Biotechnologies, Inc.), pTDT1 (ATCC, #31255), pRc/CMV (Invitrogen, Inc.), the preferred vector described in the Examples, and the like eukaryotic expression vectors.

A particularly preferred system for gene expression includes a gene delivery component, that is, the ability to deliver the gene to the tissue of interest. Suitable vectors are "infectious" vectors such as recombinant DNA viruses, adenovirus or retrovirus vectors which are engineered to express the desired protein and have features which allow infection of preselected target tissues. Particularly preferred is the replication competent avian sarcoma virus (RCAS) described herein.

Mammalian cell systems that utilize recombinant viruses or viral elements to direct expression may be engineered. For example, when using adenovirus expression vectors, the coding sequence of a polypeptide may be ligated to an adenovirus transcription/ translation control complex, e.g., the late promoter and tripartite leader sequence. This chimeric gene may then be inserted into the adenovirus genome by in vitro or in vivo recombination. Insertion in a non-essential region of the viral genome (e.g., region E1 or E3) will result in a recombinant virus that is viable and capable of expressing the polypeptide in infected hosts (e.g., see Logan et al., Proc. Natl. Acad. Sci., USA, 81:3655-3659 (1984)). Alternatively, the vaccinia virus 7.5K promoter may be used. (e.g., see, Mackett et al., Proc. Natl. Acad. Sci., USA, 79:7415-7419 (1982); Mackett et al., J. Virol., 49:857-864 (1984); Panicali et al., Proc. Natl. Acad. Sci., USA, 79:4927-4931 (1982)). Of particular interest are vectors based on bovine papilloma virus which have the ability to replicate as extrachromosomal elements (Sarver et al., Mol. Cell. Biol., 1:486 (1981)). Shortly after entry of this DNA into target cells, the plasmid replicates to about 100 to 200 copies per cell. Transcription of the inserted cDNA does not require integration of the plasmid into the host's chromosome, thereby yielding a high level of expression. These vectors can be used for stable expression by including a selectable marker in the plasmid, such as the neo gene. Alternatively, the retroviral genome can be modified for use as a vector capable of introducing and directing the expression of the polypeptide-encoding nucleotide sequence in host cells (Cone et al., Proc. Natl. Acad. Sci., USA, 81:6349-6353 (1984)). High level expression may also be achieved using inducible promoters, including, but not limited to, the metallothionine IIA promoter and heat shock promoters.

Recently, long-term survival of cytomegalovirus (CMV) promoter versus Rous sarcoma virus (RSV) promotor-driven thymidine kinase (TK) gene therapy in nude mice bearing human ovarian cancer has been studied. Cell killing efficacy of adenovirus-mediated CMV promoter-driven herpes simplex virus TK gene therapy was found to be 2 to 10 time more effective than RSV driven therapy. (Tong et al., 1999, Hybridoma 18(1):93-97). The design of chimeric promoters for gene therapy applications, which call for low level expression followed by inducible high-level expression has also been described. (Suzuki et al., 1996, Human Gene Therapy 7:1883-1893).

For long-term, high-yield production of recombinant proteins, stable expression is preferred. Rather than using expression vectors which contain viral origins of replication, host cells can be transformed with a cDNA controlled by appropriate expression control elements (e.g., promoter and enhancer sequences, transcription terminators, polyadenylation sites, etc.), and a selectable marker. As mentioned above, the selectable marker in the recombinant plasmid confers resistance to the selection and allows cells to stably integrate the plasmid into their chromosomes and grow to form foci which in turn can be cloned and expanded into cell lines.

For example, following the introduction of foreign DNA, engineered cells may be allowed to grow for 1-2 days in an enriched media, and then are switched to a selective media. A number of selection systems may be used, including but not limited to the herpes simplex virus thymidine kinase (Wigler et al., Cell, 11:223 (1977)), hypoxanthine-guanine phosphoribosyltransferase (Szybalska et al., Proc. Natl. Acad. Sci., USA, 48:2026 (1962)), and adenine phosphoribosyltransferase (Lowy et al., Cell, 22:817 (1980)) genes, which can be employed in tk⁻, hgprt⁻ or aprt⁻ cells respectively. Also, antimetabolite resistance-conferring genes can be used as the basis of selection; for example, the genes for dhfr, which confers resistance to methotrexate (Wigler et al., Proc. Natl. Acad. Sci., USA, 77:3567 (1980); O'Hare et al., Proc. Natl. Acad. Sci., USA, 78:1527 (1981); gpt, which confers resistance to mycophenolic acid (Mulligan et al., Proc Natl. Acad. Sci., USA, 78:2072, (1981)); neo, which confers resistance to the aminoglycoside G-418 (Colberre-Garapin et al, J. Mol. Biol., 150:1 (1981)); and hygro, which confers resistance to hygromycin (Santerre et al, Gene, 30:147 (1984)). Recently, additional selectable genes have been described, namely trpB, which allows cells to utilize indole in place of tryptophan; hisD, which allows cells to utilize histinol in place of histidine (Hartman et al., Proc. Natl. Acad. Sci., USA, 85:804 (1988)); and ODC (ornithine decarboxylase) which confers resistance to the ornithine decarboxylase inhibitor, 2-(difluoromethyl)-DL-ornithine, DFMO (McConlogue L., In: Current Communications in Molecular Biology, Cold Spring Harbor Laboratory ed., (1987)).

The principal vectors contemplated for human gene therapy, are derived from retroviral origin. (Wilson, 1997, Clin. Exp. Immunol. 107 (Sup. 1):31-32; Bank et al., 1996, Bioessays 18(12):999-1007; Robbins et al., 1998, Pharmacol. Ther. 80 (1) : 35-47). The therapeutic potential of gene transfer and antisense therapy has stimulated the development of many vector systems for treating a variety of tissues. (Vasculature, Stephan et al., 1997, Fundam. Clin. Pharmacol. 11(2):97-110; Feldman et al., 1997, Cardiovasc. Res. 35(3):391-404; Vassalli et al., 1997, Cardiovasc. Res. 35(3):459-69; Baek et al., 1998, Circ. Res. 82(3):295-305; kidney, Lien et al., 1997, Kidney Int. Suppl. 61:585-8; liver, Ferry et al., 1998, Hum.Gene Ther. 9(14):1975-81; muscle, Marshall et al., 1998, Curr. Opn. Genet. Dev. 8(3):360-5). In addition to these tissues, a critical target for human gene therapy is cancer, either the tumor itself, or associated tissues. (Runnebaum, 1997, Anticancer Res. 17(4B):2887-90; Spear et al., 1998, J. Neurovirol. 4(2):133-47).

Specific examples of viral gene therapy vector systems are briefly described below. Retroviral gene delivery has been recently reviewed by Federspiel and Hughes (1998, Methods in Cell Biol. 52:179-214) which describes in particular, the avian leukosis virus (ALV) retrovirus family (Federspiel et al., Proc. Natl. Acad. Sci., USA, 93:4931 (1996); Federspiel et al., Proc. Natl. Acad. Sci., USA, 91:11241 (1994)). Retroviral vectors, including ALV and murine leukemia virus (MLV) are further described by Svoboda (1998, Gene 206:153-163).

Modified retroviral/adenoviral expression systems can be readily adapted. For example, murine leukemia virus (MLV) systems are reviewed by Karavanas et al., 1998, Crit. Rev. in Oncology/Hematology 28:7-30. Adenovirus expression systems are reviewed by Von Seggern and Nemerow in Gene Expression Systems (ed. Fernandez & Hoeffler, Academic Press, San Diego, CA, 1999, chapter 5, pages 112-157).

Protein expression systems have been demonstrated to have effective use both in vivo and in vitro. For example, efficient gene transfer to human squamous cell carcinomas by a herpes simplex virus (HSV) type 1 amplicon vector has been described. (Carew et al., 1998, Am. J. Surg. 176:404-408). Herpes simplex virus has been used for gene transfer to the nervous system. (Goins et al., 1997, J. Neurovirol. 3 (Sup. 1):S80-8). Targeted suicide vectors using HSV-TK has been tested on solid tumors. (Smiley et al., 1997, Hum. Gene Ther. 8(8):965-77). Herpes simplex virus type 1 vector has been used for cancer gene therapy on colon carcinoma cells. (Yoon et al., 1998, Ann. Surg. 228(3):366-74). Hybrid vectors have been developed to extend the length of time of transfection, including HSV/AAV (adeno-associated virus) hybrids for treating hepatocytes. (Fraefel et al., 1997, Mol. Med. 3(12):813-825).

Vaccinia virus has been developed for human gene therapy because of its large genome. (Peplinski et al., 1998, Surg. Oncol. Clin. N. Am. 7(3):575-88). Thymidine kinase-deleted vaccinia virus expressing purine nucleoside pyrophosphorylase has been described for use as a tumor directed gene therapy vector. (Puhlman et al., 1999, Human Gene Therapy 10:649-657).

Adeno-associated virus 2 (AAV) has been described for use in human gene therapy, however AAV requires a helper virus (such as adenovirus or herpes virus) for optimal replication and packaging in mammalian cells. (Snoeck et al., 1997, Exp. Nephrol. 5(6):514-20; Rabinowitz et al., 1998, Curr. Opn. Biotechnol. 9(5):470-5). However, in vitro packaging of an infectious recombinant AAV has been described, making this system much more promising. (Ding et al., 1997, Gene Therapy 4:1167-1172). It has been shown that the AAV mediated transfer of ecotropic retrovirus receptor cDNA allows ecotropic retroviral transduction of established and primary human cells. (Qing et al., 1997, J. Virology 71 (7) : 5663-5667). Cancer gene therapy using an AAV vector expressing human wild-type p53 has been demonstrated. (Qazilbash et al., 1997, Gene Therapy 4:675-682). Gene transfer into vascular cells using AAV vectors has also been shown. (Maeda et al., 1997, Cardiovascular Res. 35:514-521). AAV has been demonstrated as a suitable vector for liver directed gene therapy. (Xiao et al., 1998, J. Virol. 72(12):10222-6). AAV vectors have been demonstrated for use in gene therapy of brain tissues and the central nervous system. (Chamberlin et al., 1998, Brain Res. 793(1-2):169-75; During et al., 1998, Gene Therapy 5(6):820-7). AAV vectors have also been compared with adenovirus vectors (AdV) for gene therapy of the lung and transfer to human cystic fibrosis epithelial cells. (Teramoto et al., 1998, J. virol. 72(11):8904-12).

Chimeric AdV/retroviral gene therapy vector systems which incorporate the useful qualities of each virus to create a nonintegrative AdV that is rendered functionally integrative via the intermediate generation of a retroviral producer cell. (Feng et al., 1997, Nat. Biotechnology 15(9):866-70; Bilbao et al., 1997, FASEB J. 11(8):624-34). This powerful new generation of gene therapy vector has been adapted for targeted cancer gene therapy. (Bilbao et al., 1998, Adv. Exp. Med. Biol. 451:365-74). Single injection of AdV expressing p53 inhibited growth of subcutaneous tumor nodules of human prostrate cancer cells. (Asgari et al., 1997, Int. J. Cancer 71(3):377-82). AdV mediated gene transfer of wild-type p53 in patients with advanced non-small cell lung cancer has been described. (Schuler et al., 1998, Human Gene Therapy 9:2075-2082). This same cancer has been the subject of p53 gene replacement therapy mediated by AdV vectors. (Roth et al., 1998, Semin. Oncol. 25(3 Suppl 8):33-7). AdV mediated gene transfer of p53 inhibits endothelial cell differentiation and angiogenesis in vivo. (Riccioni et al., 1998, Gene Ther. 5(6):747-54). Adenovirus-mediated expression of melanoma antigen gp75 as immunotherapy for metastatic melanoma has also been described. (Hirschowitz et al., 1998, Gene Therapy 5:975-983). AdV facilitates infection of human cells with ecotropic retrovirus and increases efficiency of retroviral infection. (Scott-Taylor, et al., 1998, Gene Ther. 5(5):621-9). AdV vectors have been used for gene transfer to vascular smooth muscle cells (Li et al., 1997, Chin. Med. J.(Engl) 110(12):950-4), squamous cell carcinoma cells (Goebel et al., 1998, Otolarynol. Head Neck Surg.119(4):331-6), esophageal cancer cells (Senmaru et al., 1998, Int_J. Cancer 78(3):366-71), mesangial cells (Nahman et al., 1998, J. Investig. Med. 46(5) :204-9), glial cells (Chen et al., 1998, Cancer Res. 58(16):3504-7), and to the joints of animals (Ikeda et al., 1998, J. Rheumatol. 25(9):1666-73). More recently, catheter-based pericardial gene transfer mediated by AcV vectors has been demonstrated. (March et al., 1999, Clin. Cardiol. 22(1 Suppl 1):I23-9). Manipulation of the AdV system with the proper controlling genetic elements allows for the AdV-mediated regulable target gene expression in vivo. (Burcin et al., 1999, PNAS (USA) 96(2):355-60).

Alphavirus vectors have been developed for human gene therapy applications, with packaging cell lines suitable for transformation with expression cassettes suitable for use with Sindbis virus and Semliki Forest virus-derived vectors. (Polo et al., 1999, Proc. Natl. Acad. Sci., USA, 96:4598-4603). Noncytopathic flavivirus replicon RNA-based systems have also been developed. (Varnavski et al., 1999, Virology 255(2):366-75). Suicide HSV-TK gene containing sinbis virus vectors have been used for cell-specific targeting into tumor cells. (Iijima et al., 1998, Int. J. Cancer 80(1):110-8).

Retroviral vectors based on human foamy virus (HFV) also show promise as gene therapy vectors. (Trobridge et al., 1998, Human Gene Therapy 9:2517-2525). Foamy virus vectors have been designed for suicide gene therapy. (Nestler et al., 1997, Gene Ther. 4(11):1270-7). Recombinant murine cytomegalovirus and promoter systems have also been used as vectors for high level expression. (Manning et al., 1998, J. Virol. Meth. 73(1):31-9; Tong et al., 1998, Hybridoma 18(1):93-7).

Gene delivery into non-dividing cells has been made feasible by the generation of Sendai virus based vectors. (Nakanishi et al., 1998, J. Controlled Release 54(1):61-8).

In other efforts to enable the transformation of non-dividing somatic cells, lentiviral vectors have been explored. Gene therapy of cystic fibrosis using a replication-defective human immunodeficiency virus (HIV) based vector has been described. (Goldman et al., 1997, Human Gene Therapy 8:2261-2268). Sustained expression of genes delivered into liver and muscle by lentiviral vectors has also been shown. (Kafri et al., 1997, Nat. Genet. 17(3):314-7). However, safety concerns are predominant, and improved vector development is proceeding rapidly. (Kim et al., 1998, J. Virol. 72(2):994-1004). Examination of the HIV LTR and Tat yield important information about the organization of the genome for developing vectors. (Sadaie et al., 1998, J. Med. Virol. 54(2):118-28). Thus the genetic requirements for an effective HIV based vector are now better understood. (Gasmi et al., 1999, J. Virol. 73(3):1828-34). Self inactivating vectors, or conditional packaging cell lines have been described. (for example Zuffery et al., 1998, J. Virol. 72(12):9873-80; Miyoshi et al., 1998, J. Virol. 72(10):8150-7; Dull et al., 1998, J. Virol. 72(11):8463-71; and Kaul et al., 1998, Virology 249(1):167-74). Efficient transduction of human lymphocytes and CD34+ cells by HIV vectors has been shown. (Douglas et al., 1999, Hum. Gene Ther. 10(6):935-45; Miyoshi et al., 1999, Science 283(5402):682-6). Efficient transduction of nondividing human cells by feline immunodeficiency virus (FIV) lentiviral vectors has been described, which minimizes safety concerns with using HIV based vectors. (Poeschla et al., 1998, Nature Medicine 4(3):354-357). Productive infection of human blood mononuclear cells by FIV vectors has been shown. (Johnston et al., 1999, J. Virol. 73(3):2491-8).

While many viral vectors are difficult to handle, and capacity for inserted DNA limited, these limitations and disadvantages have been addressed. For example, in addition to simplified viral packaging cell lines, Miniviral vectors, derived from human herpes virus, herpes simplex virus type 1 (HSV-1), and Epstein-Barr virus (EBV), have been developed to simplify manipulation of genetic material and generation of viral vectors. (Wang et al., 1996, J. Virology 70(12):8422-8430). Adaptor plasmids have been previously shown to simplify insertion of foreign DNA into helper-independent Retroviral vectors. (1987, J. Virology 61(10):3004-3012).

Viral vectors are not the only means for effecting gene therapy, as several non-viral vectors have also been described. A targeted non-viral gene delivery vector based on the use of Epidermal Growth Factor/DNA polyplex (EGF/DNA) has been shown to result in efficient and specific gene delivery. (Cristiano, 1998, Anticancer Res. 18:3241-3246). Gene therapy of the vasculature and CNS have been demonstrated using cationic liposomes. (Yang et al., 1997, J. Neurotrauma 14(5):281-97). Transient gene therapy of pancreatitis has also been accomplished using cationic liposomes. (Denham et al., 1998, Ann. Surg. 227(6):812-20). A chitosan-based vector/DNA complexes for gene delivery have been shown to be effective. (Erbacher et al., 1998, Pharm. Res. 15(9):1332-9). A non-viral DNA delivery vector based on a terplex system has been described. (Kim et al., 1998, 53(1-3):175-82). Virus particle coated liposome complexes have also been used to effect gene transfer. (Hirai et al., 1997, Biochem. Biophys. Res. Commun. 241 (1) :112-8).

Gene therapy by direct tumor injections of nonviral T7 vector encoding a thymidine kinase gene has been demonstrated. (Chen et al., 1998, Human Gene Therapy 9:729-736). Plasmid DNA preparation is important for direct injection gene transfer. (Horn et al., 1995, Hum. Gene Ther. 6(5):656-73). Modified plasmid vectors have been adapted specifically for direct injection. (Hartikka et al., 1996, Hum. Gene Ther. 7(10):1205-17).

Thus, a wide variety of gene transfer/gene therapy vectors and constructs are known in the art. By the appropriate manipulation using recombinant DNA/molecular biology techniques to insert an operatively linked Src or Yes, or both (either active or inactive) into the selected expression/delivery vector, many equivalent vectors can be generated.

### E. Modulation of Vascular Permeability (VP)

The invention provides for a composition for use in a method for the modulation of vascular permeability (VP) of the blood vessels in a tissue associated with a disease process or condition, and thereby effect events in the tissue which depend upon VP. Generally, the method comprises administering to the tissue, associated with a disease process or condition, a composition comprising a VP-modulating amount of a Yes protein, or both Src and Yes Protein.

As described herein, any of a variety of tissues, or organs comprised of organized tissues, can be a location for VP in disease conditions including brain, skin, muscle, gut, connective tissue, joints, bones and the like tissue in which blood vessels are present.

The patient treated in the present invention in its many embodiments is desirably a human patient, although it is to be understood that the principles of the invention indicate that the invention is effective with respect to all mammals, which are intended to be included in the term "patient". In this context, a mammal is understood to include any mammalian species in which treatment of tissue associated with diseases involving angiogenesis is desirable, particularly agricultural and domestic mammalian species.

Thus, the method comprises administering to a patient a therapeutically effective amount of a physiologically tolerable composition containing a Yes protein, or both Src and Yes protein.

The dosage ranges for the administration of a Src or Yes protein depend upon the form of the protein, and its potency, as described further herein, and are amounts large enough to produce the desired effect in which VP and the disease symptoms mediated by VP are ameliorated. The dosage should not be so large as to cause adverse side effects, such as hyperviscosity syndromes, pulmonary edema, congestive heart failure, and the like. Generally, the dosage will vary with the age, condition, sex and extent of the disease in the patient and can be determined by one of skill in the art. The dosage can also be adjusted by the individual physician in the event of any complication.

A therapeutically effective VP modulating amount is an amount of Src or Yes protein or mixture thereof sufficient to produce a measurable modulation of VP in the tissue being treated, i.e., a VP-modulating amount. Modulation of VP can be measured by assay as described herein, or by other methods known to one skilled in the art. Modulation of VP can be measured by the Miller assay, as described herein, or by other methods known to one of skill in the art.

The Src or Yes protein can be administered parenterally by injection or by gradual infusion over time. Although the tissue to be treated can typically be accessed in the body by systemic administration and therefore most often treated by intravenous administration of therapeutic compositions, other tissues and delivery means are contemplated where there is a likelihood that the tissue targeted contains the target molecule. Thus, compositions of the invention can be administered intravenously, intraperitoneally, intramuscularly, subcutaneously, intracavity, transdermally, and can be delivered by peristaltic means.

The therapeutic compositions containing a Src or Yes protein can be conventionally administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; i.e., carrier, or vehicle.

In one preferred embodiment the reagent is administered in a single dosage intravenously. Localized administration can be accomplished by direct injection or by taking advantage of anatomically isolated compartments, isolating the microcirculation of target organ systems, reperfusion in a circulating system, or catheter based temporary occlusion of target regions of vasculature associated with diseased tissues.

The compositions are administered in a manner compatible with the dosage formulation, and in a therapeutically effective amount. The quantity to be administered and timing depends on the subject to be treated, capacity of the subject's system to utilize the active ingredient, and degree of therapeutic effect desired. Precise amounts of active ingredient required to be administered depend on the judgement of the practitioner and are peculiar to each individual. However, suitable dosage ranges for systemic application are disclosed herein and depend on the route of administration. Suitable regimes for administration are also variable, but are typified by an initial administration followed by repeated doses at one or more hour intervals by a subsequent injection or other administration. Alternatively, continuous intravenous infusion sufficient to maintain concentrations in the blood in the ranges specified for in vivo therapies are contemplated.

There are a variety of diseases in which inhibition of angiogenesis is believed to be important, referred to as angiogenic diseases, including but not limited to, inflammatory disorders such as immune and non-immune inflammation, chronic articular rheumatism and psoriasis, disorders associated with inappropriate or inopportune invasion of vessels such as diabetic retinopathy, neovascular glaucoma, restenosis, capillary proliferation in atherosclerotic plaques and osteoporosis, and cancer associated disorders, such as solid tumors, solid tumor metastases, angiofibromas, retrolental fibroplasia, hemangiomas, Kaposi sarcoma and the like cancers which require neovascularization to support tumor growth.

Similarly, vascular permeability is an important component of angiogenesis, and in its own right associated with detrimental pathologies. For example, damage due to stroke induced vascular permeability triggers inflammation related damage.

Thus, methods which inhibit vascular permeability in a tissue associated with a disease condition ameliorates symptoms of the disease and, depending upon the disease, can contribute to cure of the disease. In one embodiment, the invention contemplates inhibition of vascular permeability, per se, in a tissue associated with a disease condition. The extent of vascular permeability in a tissue, and therefore the extent of inhibition achieved, can be evaluated by a variety of methods.

Thus, in one related embodiment, a tissue to be treated is an inflamed tissue and the vascular permeability to be inhibited is due to VEGF mediated stimulation. In this class the method contemplates inhibition of VP in arthritic tissues, such as in a patient with chronic articular rheumatism, in immune or non-immune inflamed tissues, in psoriatic tissue and the like.

In another related embodiment, a tissue to be treated is a retinal tissue of a patient with a retinal disease such as diabetic retinopathy, macular degeneration or neovascular glaucoma and the VP to be inhibited is retinal tissue VP where there is neovascularization of retinal tissue.

The methods are also particularly effective against the formation of metastases because (1) their formation requires vascularization of a primary tumor so that the metastatic cancer cells can exit the primary tumor and (2) their establishment in a secondary site requires neovascularization to support growth of the metastases.

In a related embodiment, the invention contemplates the practice in conjunction with other therapies such as conventional chemotherapy directed against solid tumors and for control of establishment of metastases. The administration of VP inhibitor is typically conducted during or after chemotherapy, although it is preferably to inhibit VP after a regimen of chemotherapy at times where the tumor tissue will be responding to the toxic assault by inducing VP to recover by the provision of a blood supply and nutrients to the tumor tissue. In addition, it is possible to administer the vascular permeability inhibition methods after surgery where solid tumors have been removed as a prophylaxis against metastases.

Insofar as the present invention applies to inhibition vascular permeability involved with metastases, the invention can also apply to inhibition of metastases formation, and to regression of established tumors.

Restenosis is a process of smooth muscle cell (SMC) migration and proliferation into the tissue at the site of percutaneous transluminal coronary angioplasty which hampers the success of angioplasty. The migration and proliferation of SMC's during restenosis can be considered a process of VP which is inhibited according to the invention. Therefore, the invention also contemplates inhibition of restenosis by inhibiting vascular permeability in a patient following angioplasty procedures. For inhibition of restenosis, the inactivated tyrosine kinase is typically administered after the angioplasty procedure because the coronary vessel wall is at risk of restenosis, typically for from about 2 to about 28 days, and more typically for about the first 14 days following the procedure.

The composition of the invention can be used in a method for inhibiting vascular permeability in a tissue associated with a disease condition, and therefore for also practicing the treatment of vascular permeability-related diseases, comprising contacting a tissue in which increased vascular permeability is occurring, or is at risk for occurring, with a composition comprising a therapeutically effective amount of an inactivated Yes protein or an inactivaved Src and Yes protein.

In cases where it is desirable to promote or potentiate VP, administration of an active Yes protein or an active Src and Yes protein to the tissue is useful. The routes and timing of administration are comparable to the methods described hereinabove for inhibition.

For example, manipulation of the permeability of the blood-brain barrier to modulate the access of drugs to the brain tissue is contemplated. An increase in vascular permeability of the blood-brain barrier will allow for drugs, that may normally not cross the barrier, to enter in to the brain tissues.

Refined modulation of angiogenesis in conjunction with VP may be desired, and thus a mixture of active and inactive forms of Src protein or Yes protein can be administered.

Inhibition or potentiation of angiogenesis clearly occurs by 5 to 7 days after the initial contacting with the therapeutic composition of the examples. Similarly, modulation of VP can occur in a similar time frame. Effects can occur within a short time after administration of the therapeutic composition. The time-limiting factors include rate of tissue absorption, cellular uptake, protein translocation or nucleic acid translation (depending on the therapeutic) and protein targeting. Thus, VP modulating effects can occur in as little as an hour from time of administration. Additional or prolonged exposure to inactive Src and/or Yes protein can also be done, utilizing the proper conditions. Thus, a variety of desired therapeutic time frames can be designed by modifying such parameters.

The method also comprises administering to a tissue associated with a disease process or blood vessel injury or trauma condition, a composition comprising a Src family tyrosine kinase inhibitor. A Src family tyrosine kinase inhibitor can be a chemical Src inhibitor, a protein Src inhibitor, or a nucleic acid Src inhibitor.

Examples of suitable chemical Src family tyrosine kinase inhibitors include and are not limited to PP1, PP2, PD173955, AGL1872, PD162531, Radicicol R2146, Geldanamycin and the like.

PP1 (from Biomol, by license from Pfizer), was the synthetic Src inhibitor used for these studies. PP1 is part of the pyrazolopyrimidine family of Src inhibitors. Other synthetic Src inhibitors include PP2 (from Calbiochem, on license from Pfizer) which is related in structure to PP1 and has also been shown to block Src family kinase activity. (Hanke et al., 1996, J. Biol. Chem. 271(2): 695-701). Other specific Src kinase inhibitors include PD173955 (Moasser et al., 1999, Cancer Res. 59:6145-6152; Parke Davis) for which the structure has been published. PD162531 (Owens et al., 2000, Mol. Biol. Cell 11:51-64) is also a specific Src kinase inhibitor from Parke Davis but the structure is not accessible from the literature. Geldanamycin is also a Src kinase inhibitor, available from Life Technologies. Radicicol, which is offered commercially by different companies (e.g. Calbiochem, RBI, Sigma), is an antifungal macrocyclic lactone antibiotic that also acts as an unspecific protein tyrosine kinase inhibitor and was shown to inhibit Src kinase activity. Preferred chemical inhibitors are PP1 and PP2 or the like, a most preferred chemical inhibitor being PP1.

Additional suitable Src family tyrosine kinase inhibitors can be identified and characterized using standard assays known in the art. For example, screening of chemical compounds for potent and selective inhibitors for Src or other tyrosine kinases has been done and have resulted in the identification of chemical moieties useful in potent inhibitors of Src family tyrosine kinases.

For example, catechols have been identified as important binding elements for a number of tyrosine kinase inhibitors derived from natural products, and have been found in compounds selected by combinatorial target-guided selection for selective inhibitors of c-Src. Maly, D.J., et al. (2000, "Combinatorial target-guided ligand assembly: Identification of potent subtype-selective c-Src inhibitors" PNAS (USA) 97(6): 2419-2424). Combinatorial chemistry based screening of candidate inhibitor compounds, using moieties known to be important to Src inhibition as a starting point, is a potent and effective means for isolating and characterizing other chemical inhibitors of Src family tyrosine kinases.

However, even careful selection of potential binding elements based upon the potential for mimicking a wide range of functionalities present on polypeptides and nucleic acids can be used to perform combinatorial screens for active inhibitors. For example, O-methyl oxime libraries are particularly suited for this task, given that the library is easily prepared by condensation of O-methylhydroxylamine with any of a large number of commercially available aldehydes. O-alkyl oxime formation is compatible with a wide range of functionalities which are stable at physiological pH. Malay et al., supra.

As described, suitable Src family kinase inhibitors also include VP-inhibiting amount of an inactive Src or Yes protein, or mixture thereof.

Other suitable Src family kinase inhibitors include CSK, or nucleic acid vector expressing inactivating amounts of CSK.

As described herein, any of a variety of tissues, or organs comprised of organized tissues, can be a location for VP in disease conditions including brain, skin, muscle, gut, connective tissue, joints, bones and the like tissue in which blood vessels are present.

The patient that can be treated according to the present invention is desirably a human patient, although it is to be understood that the principles of the invention indicate that the treatment is effective with respect to all mammals. In this context, a mammal is understood to include any mammalian species in which treatment of vascular leakage or edema associated tissue damage is desirable, particularly agricultural and domestic mammalian species.

The dosage ranges for the administration of chemical Src family tyrosine kinase inhibitors, such as PP1 can be in the range of about .1 mg/kg body weight to about 10 mg/kg body weight, or the limit of solubility of the active agent in the pharmaceutical carrier. Preferably, typical dosages can be from about 1 mg/kg body weight to about 9 mg/kg body weight. Lower dosages, such as from 1 mg/kg body weight to about 1 mg/kg body weight can be optimized for multiple administration to treat chronic conditions. Typical dosages for treating acute conditions that are less severe, easily accessible, and where the route of administration is more direct, can be from about 1 mg/kg body weight to about 3 mg/kg body weight. Depending upon the severity of the injury, location, or the route of administration, a higher dose of from about 3 mg/kg body weight to 10 mg/kg body weight (or limit of solubility of the agent in the pharmaceutical carrier) may be used when encountering a more severe injury, hard to access location, or where administration can only be via indirect systemic route.

In the case of acute injury or trauma, it is best to administer treatment as soon as possible after the occurrence of the incident. However, time for effective administration of a Src family tyrosine kinase inhibitors can be within about 48 hours of the onset of injury or trauma, in the case of acute incidents. It is preferred that administration occur within about 24 hours of onset, within 12 hours being better, and most preferred that administration take place within about 6 hours of onset. Administration after 48 hours of initial injury may be appropriate to ameliorate additional tissue damage due to further vascular leakage or edema, however the effect on the initial tissue damage may be greatly reduced.

Where prophylactic administration is made to prevent vascular leakage or edema associated with surgical procedure, or made in view of predisposing diagnostic criteria, administration can occur prior to any actual VP increase, or during such VP increase causing event. For the treatment of chronic conditions which lead to VP increase and associated vascular leaking or edema, administration of active Src family tyrosine kinase inhibitors can be made with a continuous dosing regimen.

The dosage ranges for the administration of an inactive Src or Yes protein, or active CSK protein depend upon the form of the protein, and its potency, as described further herein, and are amounts large enough to produce the desired effect in which VP and the disease symptoms mediated by VP are ameliorated. The dosage should not be so large as to cause adverse side effects, such as hyperviscosity syndromes, pulmonary edema, congestive heart failure, and the like.

A therapeutically effective VP modulating amount is an amount of active CSK or inactive Src or Yes protein, or mixture thereof, sufficient to produce a measurable modulation of VP in the tissue being treated, ie., a VP-modulating amount. Modulation of VP can be measured by assay as described herein, or by other methods known to one skilled in the art. Modulation of VP can be measured by the Miller assay, as described herein, or by other methods known to one of skill in the art.

Generally, the dosage can vary with the age, condition, sex and extent of the disease in the patient and can be determined by one of skill in the art. The dosage can also be adjusted by the individual physician in the event of any complication.

The pharmaceutical compositions of the invention can be administered parenterally by injection or by gradual infusion over time. Although the tissue to be treated can typically be accessed in the body by systemic administration and therefore most often treated by intravenous administration of therapeutic compositions, other tissues and delivery means are contemplated where there is a likelihood that the tissue targeted contains the target molecule. Thus, compositions of the invention can be administered intravenously, intraperitoneally, intramuscularly, subcutaneously, intracavity, transdermally, and can be delivered by peristaltic means.

Intravenous administration is effected by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; i.e., carrier, or vehicle.

In one preferred embodiment the active agent is administered in a single dosage intravenously. Localized administration can be accomplished by direct injection or by taking advantage of anatomically isolated compartments, isolating the microcirculation of target organ systems, reperfusion in a circulating system, or catheter based temporary occlusion of target regions of vasculature associated with diseased tissues.

The compositions are administered in a manner compatible with the dosage formulation, and in a therapeutically effective amount. The quantity to be administered and timing depends on the subject to be treated, capacity of the subject's system to utilize the active ingredient, and degree of therapeutic effect desired. Precise amounts of active ingredient to be administered depend on the judgement of the practitioner and are peculiar to each individual. However, suitable dosage ranges for systemic application are disclosed herein and depend on the route of administration. Suitable regimes for administration are also variable, but are typified by an initial administration followed by repeated doses at one or more hour intervals by a subsequent injection or other administration. Alternatively, continuous intravenous infusion sufficient to maintain concentrations in the blood in the ranges specified for in vivo therapies are contemplated.

Ameliorating tissue damage due to vascular leakage or edema associated with a disease condition, injury or trauma ameliorates symptoms of the disease and, depending upon the disease, can contribute to cure of the disease. The extent of vascular permeability in a tissue, and therefore the extent of inhibition, can be evaluated by a variety of methods. In particular, stroke or other cerebrovascular accident related injury to the CNS that occur due to injury induced increase of VP, and subsequent vascular leakage and/or edema damage to associated tissues, can be ameliorated.

In one related embodiment, a tissue to be treated is an inflamed tissue and the vascular permeability to be inhibited is due to VEGF mediated stimulation. For this type of affliction, the invention contemplates inhibition of VP in arthritic tissues, such as in a patient with chronic articular rheumatism, in immune or non-immune inflamed tissues, in psoriatic tissue, and the like.

In another related embodiment, a tissue to be treated is a retinal tissue of a patient with a retinal disease such as diabetic retinopathy, macular degeneration or neovascular glaucoma and the VP to be inhibited is retinal tissue VP where there is neovascularization of retinal tissue.

The composition of the invention can be used in a method for inhibiting vascular permeability in a tissue associated with a injury or disease condition, and therefore for also practicing the treatment of vascular permeability-related diseases, comprising contacting a tissue in which increased vascular permeability is occurring, or is at risk for occurring, with a composition comprising a therapeutically effective amount of a Src family tyrosine kinase inhibitor.

Modulation of VP, and amelioration of tissue damage due to vascular leakage and edema can occur within a short time after administration of the therapeutic composition. Most therapeutic effects can be visualized within 3 days of administration, in the case of acute injury or trauma. Typically, effects of chronic administration will not be as readily apparent.

The time-limiting factors include rate of tissue absorption, cellular uptake, protein translocation or nucleic acid translation (depending on the therapeutic) and protein targeting. Thus, tissue damage modulating effects can occur in as little as an hour from time of administration of the inhibitor. Additional or prolonged exposure to Src family tyrosine kinase inhibitors can also be done, utilizing the proper conditions. Thus, a variety of desired therapeutic time frames can be designed by modifying such parameters.

### F. Therapeutic Compositions (General Considerations)

The present invention contemplates therapeutic compositions useful for practicing the therapeutic methods described herein. In a preferred embodiment, the therapeutic composition is not immunogenic when administered to a mammal or human patient for therapeutic purposes.

The Src and Yes protein can be active, inactive, or a mixture thereof depending upon the desired modulation. Preferred forms of Src and Yes are described above. The CSK protein comprises the active form.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The preparation of a pharmacological composition that contains active ingredients dissolved or dispersed therein is well understood in the art and need not be limited based on formulation. Typically such compositions are prepared as injectable either as liquid solutions or suspensions, however, solid forms suitable for solution, or suspensions, in liquid prior to use can also be prepared. The preparation can also be emulsified or presented as a liposome composition.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The therapeutic composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and organic bases such as isopropylamine, trimethylamine, 2-ethylamino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes.

Liquid compositions can also contain liquid phases in addition to and to the exclusion of water. Exemplary of such additional liquid phases are glycerin, vegetable oils such as cottonseed oil, and water-oil emulsions.

### F(i). VP Modulating Therapeutic Compositions of the Present Invention

In one embodiment of the present invention, a therapeutic composition contains a vascular permeability-modulating amount of a Yes protein or a Src and Yes protein, typically formulated to contain an amount of at least 0.1 weight percent of Src or Yes protein per weight of total therapeutic composition. A weight percent is a ratio by weight of Src or Yes protein to total composition. Thus, for example, 0.1 weight percent is 0.1 grams of Src or Yes protein per 100 grams of total composition.

### F(ii). Src Family Tyrosine Kinase Inhibitor Therapeutic Compositions of the Present Invention

Suitable Src family tyrosine kinase inhibitors will specifically inhibit the biological tyrosine kinase activity of Src family tyrosine kinases. A most suitable Src family tyrosine kinase will have primary specificity for inhibiting the activity of the ^{pp60}Src protein, and secondarily inhibit the most closely related Src family tyrosine kinases such as Yes. Examples of particularly suitable Src family tyrosine kinase inhibitors include PP1, PP2, PD173955, AGL1872, PD162531, Radicicol R2146, Geldanamycin and the like. Additional suitable chemical Src family tyrosine kinase inhibitors can be identified and characterized using standard assays known in the art.

Mutations in Src shown to be inhibiting VP instead of stimulating it, are referred to as inactive Src mutations. Proteins having mutation that confer this inhibitory activity are also referred to as dominant negative Src proteins in that they inhibit VP, including that resulting from endogenous activity of Src as well as enhanced Src activity resulting from growth factor stimulation. Thus certain mutations of wild type c-Src can also function as a dominant negative with respect to their ability to block blood vessel growth and VP, and for example, therefore decrease VP in vivo. Therefore, other suitable Src family tyrosine kinase inhibitors can include inactive forms of Src and Yes protein that can antagonize Src or Yes activity, resulting in inhibition or decrease in vascular permeability of the blood vessels in the target tissue. A preferred inactive Src protein is Src 251. Another preferred inactive Src protein is Src K295M. A preferred inactive Yes protein will have diminished kinase activity as compared with the wild-type protein.

Other Src family tyrosine kinase inhibitors can be antisense nucleic acids, nucleic acid analogs, or protein nucleic acids which inhibit the expression of Src or Yes genes in targeted cells. The antisense molecules can be a therapeutically effective VP modulating amount when said antisense nucleic acid, capable of hybridizing to the mRNA encoding for Src or Yes protein, can hybridize to such mRNA and result in an inhibition of cell expression of tyrosine kinase protein Src or Yes, when transfected into a target cell in a suitable pharmaceutical carrier.

As described, preferred inhibitory c-Src protein includes the Src 251 in which only the first 251 amino acids of Src are expressed. This construct lacks the entire kinase domain and is therefore referred to as "kinase dead" Src protein. A second construct is the Src (K295M) mutation in which the lysine amino acid residue 295 is mutated into a methionine. This point mutation in the kinase domain prevents ATP binding and also blocks kinase-dependent Src functions related to vascular cell and tumor cell signaling and proliferation.

With respect to the point mutations, any mutation resulting in the desired inhibitory activity is contemplated for use in this invention. Fusion protein constructs combining the desired Src protein (mutation or fragment thereof) with expressed amino acid tags, antigenic epitopes, fluorescent protein, or other such protein or peptides are also contemplated, so long as the desired modulating effect of the Src protein is intact.

Similarly, addition of an exogenous inhibitor of Src protein activity or the stimulation of expression of such inhibitor within the targeted tissues, such as CSK (C-terminal Src Kinase), is also a means for inhibiting Src activity. Phosphorylation of tyrosine inactivating Src, is a means for negative regulation by the c-terminal Src kinase, referred to as CSK. (Nada et al., 1991, Nature 351: 69-72; Okada et al., 1991, J. Biol. Chem. 266(36): 24249-24252). When CSK phosphorylates aa527 in the wild-type Src, the Src protein is inactivated. Thus, CSK is a useful and potent inhibitor of Src activity. Human CSK protein sequence of 450 amino acids is identified by accession number P41240 and can be found in the swiss protein data base. A human CSK encoding mRNA nucleic acid sequence is identified by accession number NM 004383 in the GenBank database.

In one embodiment of the invention, a pharmaceutical composition contains a vascular permeability-modulating amount of a Src, Yes and CSK protein, or sufficient expression vector to express an effective amount of inactive Src, Yes, or active CSK protein, typically formulated to contain an amount of at least 0.1 weight percent of protein per weight of total pharmaceutical composition. Thus, for example, 0.1 weight percent is 0.1 grams of protein per 100 grams of total composition. Thus, an effective amount of a Src family tyrosine kinase inhibitor in a pharmaceutical composition is that amount which results in therapeutically effective modulation of Src regulated vascular permeability. A therapeutic amount of any pharmaceutical composition is one which, on its own, results in the amelioration of vascular leakage or edema related tissue damage.

### G(i) General Considerations; Article of Manufacture

As used herein, the term packaging material refers to a material such as glass, plastic, paper, foil, and the like capable of holding within fixed means a pharmaceutical agent. Thus, for example, the packaging material can be plastic or glass vials, laminated envelopes and the like containers used to contain a pharmaceutical composition including the pharmaceutical agent.

In preferred embodiments, the packaging material includes a label that is a tangible expression describing the contents of the article of manufacture and the use of the pharmaceutical agent contained therein.

### G(ii) VP Modulating Therapeutic Compositions; Article of Manufacture

The invention contemplates an article of manufacture comprising a labeled container for providing a therapeutically effective amount of a mixture of Src protein and Yes protein. An article of manufacture comprises packaging material and a pharmaceutical agent contained within the packaging material.

The pharmaceutical agent in an article of manufacture is any of the compositions of the present invention suitable for providing a Src and Yes protein, formulated into a pharmaceutically acceptable form as described herein according to the disclosed indications. The article of manufacture contains an amount of pharmaceutical agent sufficient for use in treating a condition indicated herein, either in unit or multiple dosages.

The Src or Yes protein can be active or inactive, or a mixture thereof, depending upon the level of modulation desired. Preferred forms of active and inactive Src and Yes are described above.

The packaging material comprises a label which indicates the use of the pharmaceutical agent contained therein, e.g., for treating conditions assisted by the inhibition or potentiation of vascular permeability, and the like conditions disclosed herein. The label can further include instructions for use and related information as may be required for marketing. The packaging material can include container(s) for storage of the pharmaceutical agent.

### G(iii). Src Family Tyrosine Kinase Inhibitor Composition; Article of Manufacture

The invention also contemplates an article of manufacture which is a labeled container for providing a therapeutically effective amount of a Src family tyrosine kinase inhibitor. The inhibitor may be a packaged chemical, protein or nucleic acid Src family tyrosine kinase inhibitor, combinations of more than one, or mixtures thereof. An article of manufacture comprises packaging material and a pharmaceutical agent contained within the packaging material. The article of manufacture may also contain two or more sub-therapeutically effective amounts of a pharmaceutical composition, which together act synergistically to result in amelioration of tissue damage due to vascular leakage or edema.

The pharmaceutical agent in an article of manufacture is any of the compositions of the present invention suitable for providing a Src family tyrosine kinase inhibitor, formulated into a pharmaceutically acceptable form as described herein according to the disclosed indications. Thus, the composition can comprise a chemical inhibitory such as PP1, PP2, PD173955, AGL1872, PD162531, Radicicol R2146, and Geldanamycin, a protein inhibitor such as inactive Src, inactive Yes, active CSK protein, or a nucleic acid molecule which is capable of expressing such protein or combination of proteins. The article of manufacture contains an amount of pharmaceutical agent sufficient for use in treating a condition indicated herein, either in unit or multiple dosages.

The packaging material comprises a label which indicates the use of the pharmaceutical agent contained therein, e.g., for treating conditions assisted by the inhibition of vascular permeability increase, and the like conditions disclosed herein. The label can further include instructions for use and related information as may be required for marketing. The packaging material can include container(s) for storage of the pharmaceutical agent.

### Examples

The following examples relating to this invention are illustrative and should not, of course, be construed as specifically limiting the invention. Moreover, such variations of the invention, now known or later developed, which would be within the purview of one skilled in the art are to be considered to fall within the scope of the present invention hereinafter claimed.

### 1. Preparation of c-src or c-yes Expression Constructs

For preparing the expression constructs useful in modulating VP and angiogenesis, c-src cDNA is manipulated and inserted into an expression construct/vector.

The cDNA sequence encoding for wild-type (i.e., endogenous) chicken c-src is shown in FIG. 1 (SEQ ID NO:2) with the encoded amino acid residue sequence shown in FIG. 2 (SEQ ID NO:3). The encoded protein sequence is translated from the cDNA nucleotide positions 112 to 1713. The nucleic acid sequence corresponding to the nucleic acid sequence of human c-src cDNA (SEQ ID NO:4) and encoded amino acid residue (SEQ ID NO:5) sequences are shown respectively in FIGs. 3 and 4. For the human protein sequence, the coding sequence begins at nucleotide position 134 to 1486 of the cDNA.

Wild-type as well as a number of mutated c-src cDNAs were prepared. Mutated c-Src constructs were prepared by site-directed mutagenesis as described by Kaplan et al., EMBO J., 13:4745-4756 (1994). The mutated c-Src constructs for encoding mutated Src proteins are described in Kaplan et al., id.. Kaplan et al. describe various mutated c-Src constructs and encoded proteins useful for the practice of this invention. For example, Kaplan et al. depict several products of chicken c-src alleles in their FIG. 1, including SrcA and Src251.

The present invention describes two categories of c-Src function to modulate VP. As previously discussed, one category contains Src molecules that increase VP and thus are considered to be active proteins. Wild-type Src along with various mutations are shown in the present invention to induce VP. One mutation of wild type c-src which functions in this context with respect to its ability to induce blood vessel growth and VP is the Src A mutant having a point mutation at amino acid (aa) residue position 527 changing tyrosine 527 to phenylalanine. This site is normally a site for negative regulation by the c-Src kinase, referred to as kinase CSK. When CSK phosphorylates aa527 in the wild-type Src, the protein is inactivated. However, in mutated Src A at aa527, the regulatory tyrosine converted to phenylalanine thus conferring upon the protein a constitutively (i.e., permanently) active protein not subject to inactivation by phosphorylation.

Other mutations in Src are herein shown to have the opposite modulatory effect on VP, inhibiting VP instead of stimulating it. Such mutations are referred to as inactive Src mutations. Proteins having mutation that confer this inhibitory activity are also referred to as dominant negative Src proteins in that they inhibit VP, including that resulting from endogenous activity of Src as well as enhanced Src activity resulting from growth factor stimulation. Thus certain mutations of wild type c-src can also function as a dominant negative with respect to their ability to block blood vessel growth and VP, and for example, therefore decrease VP in vivo.

Such preferred inhibitory c-Src protein includes the Src 251 in which only the first 251 amino acids of Src are expressed. This construct lacks the entire kinase domain and is therefore referred to as "kinase dead" Src protein. A second construct is the Src (K295M) mutation in which the lysine amino acid residue 295 is mutated into a methionine. This point mutation in the kinase domain prevents ATP binding and also blocks kinase-dependent Src functions related to vascular cell and tumor cell signaling and proliferation.

With respect to the point mutations, any mutation resulting in the desired inhibitory or stimulatory activity is contemplated for use in this invention. Fusion protein constructs combining the desired Src protein (mutation or fragment thereof) with expressed amino acid tags, antigenic epitopes, fluorescent protein, or other such protein or peptides are also contemplated, so long as the desired modulating effect of the Src protein is intact.

For example, for the activating mutation of Src at residue 527, as long as the resultant mutated amino acid residue is not tyrosine, serine, or threonine, the present invention contemplates that the presence of an alternate amino acid at the desired position will result in a Src protein with a desired active, VP promoting modulatory activity.

Src Family kinase Yes has been previously described, but not much has been known about its cellular function. (Burck et al., 1988, The Oncogenes, springer-Verlag, New York, pp. 133-155; Marth et al., 1985, Cell, 43:393; Semba et al., 1986, PNAS (USA) 83:5459; Shibuya et al., 1982, J. Virol. 42:143; Yoshida et al., 1985, Jpn. J. Cancer Res. 76:559). Preferred active human Yes protein is encoded for by nucleic acid described in Sukegawa et al. (1987, Mol. Cell Biol. 7:41-47). Inactivating modifications to human Yes protein and nucleic acids encoding Yes can be accomplished as described for Src.

**TABLE I**

| Src/Mutation | Src Function | | Effect on VP and Angiogenesis |
|---|---|---|---|
| c-Src | + | active | stimulates |
| SrcA (T527F) | + | active | stimulates |
| Src527 (point) | + | active | stimulates |
| Src251 | - | inactive | inhibits |
| Src (truncate) | - | inactive | inhibits |
| Src (K295M) | - | inactive | inhibits |
| Src295 (point) | - | inactive | inhibits |

One preferred expression construct is the RCASBP (A) construct (SEQ ID NO:1). This expression vector is based on a series of replication competent avian sarcoma viruses with an enhanced Bryan polymerase (BP) for improved titre, and is specific for the A type envelope glycoprotein expressed on normal avian cells (reviewed in Methods in Cell Biology, 52:179-214 (1997); see also, Hughes et al., 1987, J. Virol. 61:3004-3012; Fekete & Cepko, 1993, Mol. Cellular Biol. 13(4):2604-2613; Itoh et al., 1996, Development 122:291-300; and Stott et al., 1998, BioTechniques 24:660-666). The complete sequence of RCASBP(A) (SEQ ID NO:1) is given in the sequence listing, and a restriction map of the construct is depicted as FIG. 10, referred to herein as RCAS.

The original Src 251 construct was subcloned by Dr. Pam Schwartzberg, at NIH in Dr. Harold Varmus' laboratory. Briefly, cloning of a src cDNA sequence for expression thereof was accomplished by inserting a linker containing Not I-BstB1-Not I restriction sites into a unique Not I site in the 5' end of Src 251. Src has a unique Cla I site at the 3' end. Digestion of Src 251 with BstB1 and Cla I generated a BstB1-ClaI fragment which was then ligated into the Cla I site on RCASBP(A). A BstB1 overhang allows for ligation with a Cla I overhang that will not be recut with Cla I.

The Src constructs are readily obtained in the above vector by first digesting the RCAS vector containing Src 251 with Not I and Cla I (in a DAM+ background) to allow for insertion of a similarly digested Src cDNA. Therefore this initial RCASBP(A) construct containing Src 251 was further used to subclone all other Src constructs as described above and in Kaplan et al. (1994, The EMBO J. 13(20):4745-4756), into RCASBP(A) via a Not I-Cla I fragment generated through the Src 251 construction. To produce the desired c-src mutations in the cDNA, standard site-directed mutagenesis procedures familiar to one of ordinary skill in the art were utilized. PCR primers designed to incorporate the desired mutations were also designed with restriction sites to facilitate subsequent cloning steps. Entire segments of Src encoding nucleic acid sequences are deleted from the nucleic acid constructs through PCR amplification techniques based on the known cDNA sequences of chicken, human and the like homologs of Src and subsequent formation of new constructs.

In one embodiment, the 3' PCR primer used to amplify src nucleic acids also encodes an in-frame sequence. Use of this primer adds a 9E10-myc epitope tag to the carboxyl terminus of the subsequent Src construct. The following amino acids were added after amino acid 251 of Src to generate vector constructs containing the 9E10-myc epitope tag: VDMEQKLIAEEDLN (SEQ ID NO:6). Two separate PCRs were carried out for each construct and similar results were obtained. All mutant constructs constructed by PCR were also sequenced by PCR to confirm predicted DNA sequence of clones. Wild-type and mutated Src cDNAs for use in the expression systems are also available from Upstate Biotech Laboratories, Lake Placid, NY which sells avian as well as human src, and several kinase dead and activated mutated forms.

Alternative expression vectors for expressing the Src or Yes proteins also include adenoviral vectors as described in US Patent Numbers 4, 797, 368, 5, 173, 414, 5, 436,146, 5,589,377, and 5,670,488. Alternative methods for the delivery of the Src or Yes modulatory proteins include delivery of the Src or Yes cDNA with a non-viral vector system as described in US Patent Number 5,675,954 and delivery of the cDNA itself as naked DNA as described in US Patent Number 5,589,466. Delivery of constructs is also not limited to topical application of a viral vector as described in the CAM assay system below. For example, viral vector preparations are also injected intravenously for systemic delivery into the vascular bed. These vectors are also targetable to sites of increased neovascularization by localized injection of a tumor, as an example.

In vitro expressed proteins are also contemplated for delivery thereof following expression and purification of the selected Src protein by methods useful for delivery of proteins or polypeptides. One such method includes liposome delivery systems, such as described in US Patent Numbers 4,356,167, 5,580,575, 5,542,935 and 5,643,599. Other vector and protein delivery systems are well known to those of ordinary skill in the art for use in the expression and/or delivery of the Src or Yes proteins.

### 2. Characterization of the Untreated Chick Chorioallantoic Membrane (CAM)

### A. Preparation of the CAM

Angiogenesis can be induced on the chick chorioallantoic membrane (CAM) after normal embryonic angiogenesis has resulted in the formation of mature blood vessels. Angiogenesis has been shown to be induced in response to specific cytokines or tumor fragments as described by Leibovich et al., Nature, 329:630 (1987) and Ausprunk et al., Am. J. Pathol., 79:597 (1975). CAMs were prepared from chick embryos for subsequent induction of angiogenesis and inhibition thereof. Ten day old chick embryos were obtained from McIntyre Poultry (Lakeside, CA) and incubated at 37°C with 60% humidity. A small hole was made through the shell at the end of the egg directly over the air sac with the use of a small crafts drill (Dremel, Division of Emerson Electric Co. Racine WI). A second hole was drilled on the broad side of the egg in a region devoid of embryonic blood vessels determined previously by candling the egg. Negative pressure was applied to the original hole, which resulted in the CAM (chorioallantoic membrane) pulling away from the shell membrane and creating a false air sac over the CAM. A 1.0 centimeter (cm) x 1.0 cm square window was cut through the shell over the dropped CAM with the use of a small model grinding wheel (Dremel). The small window allowed direct access to the underlying CAM.

The resultant CAM preparation was then either used at 6 days of embryogenesis, a stage marked by active neovascularization, without additional treatment to the CAM reflecting the model used for evaluating effects on embryonic neovascularization or used at 10 days of embryogenesis where angiogenesis has subsided. The latter preparation was thus used in this invention for inducing renewed angiogenesis in response to cytokine treatment or tumor contact as described below.

### 3. CAM Angiogenesis Assay

### A. Angiogenesis Induced by Growth Factors

Angiogenesis has been shown to be induced by cytokines or growth factors. Angiogenesis was induced by placing a 5 millimeter (mm) X 5 mm Whatman filter disk (Whatman Filter paper No.1) saturated with Hanks Balanced Salt Solution (HBSS, GIBCO, Grand Island, NY) or HBSS containing 2 micrograms/milliliter (µg/ml) recombinant basic fibroblast growth factor (bFGF) or vascular endothelial cell growth factor (VEGF) (Genzyme, Cambridge, MA) on the CAM of either a 9 or 10 day chick embryo in a region devoid of blood vessels and the windows were latter sealed with tape. Other concentrations of growth factors are also effective at inducing blood vessel growth. For assays where inhibition of angiogenesis is evaluated with intravenous injections of antagonists, angiogenesis is first induced with 1-2 ug/ml bFGF or VEGF in fibroblast growth medium. Angiogenesis was monitored by photomicroscopy after 72 hours.

### B. Embryonic Angiogenesis

The CAM preparation for evaluating the effect of angiogenesis inhibitors on the natural formation of embryonic neovasculature is the 6 day embryonic chick embryo as previously described. At this stage in development, the blood vessels are undergoing de novo growth and thus provides a useful system for assessing angiogenesis modulation by the Src proteins useful in the present invention. The CAM system is prepared as described above with the exception that the assay is performed at embryonic day 6 rather than at day 9 or 10.

### 4. Modulation of Angiogenesis as Measured in the CAM Assay

To assess the effect of Src proteins on angiogenesis, the following assays were performed on 10 day old chick CAM preparations. Five µg of RCAS constructs prepared as described in Example 1 were transfected into the chicken immortalized fibroblast line, DF-1 (gift of Doug Foster, U. of Minn.). This cell line as well as primary chick embryo fibroblasts were capable of producing virus, however the DF-1 cell line produced higher titres. Viral supernatants were collected from subconfluent DF-1 producer cell lines in serum free CLM media [composition: F-10 media base supplemented with DMSO, folic acid, glutamic acid, and MEM vitamin solution]. Thirty-five ml of viral supernatant were concentrated by ultracentrifugation at 4°C for 2 hours at 22,000 rpm. These concentrated viral pellets were resuspended in 1/100 the original volume in serum-free CLM media, aliquoted and stored at -80°C. The titre was assessed by serial dilution of a control viral vector having a nucleotide sequence encoding green fluorescent protein (GFP), referred to as RCAS-GFP, infection on primary chick embryo fibroblasts that were incubated for 48-72 hours. The titres of viral stock that were obtained following concentration routinely exceeded 10⁸ I.U./ml. For the CAM assay using the viral stocks, cortisone acetate soaked Whatman filter disks 6 mm in diameter were prepared in 3 mg/ml cortisone acetate for 30 minutes in 95% ethanol. The disks were dried in a laminar flow hood and then soaked on 20 µl of viral stock per disk for 10 minutes. These disks were applied to the CAM of 9 or 10 day chick embryos and sealed with cellophane tape and incubated at 37°C for 18-24 hr. Then either mock PBS or growth factors were added at a concentration of 5 µg/ml to the CAM in a 20 µl volume of the appropriate virus stock as an additional boost of virus to the CAM tissue. After 72 hours, the CAMs were harvested and examined for changes in the angiogenic index as determined by double blind counting of the number of branch points in the CAM underlying the disk. For kinase assays, the tissue underlying the disk was harvested in RIPA, homogenized with a motorized grinder and Src immunoprecipitated from equivalent amounts of total protein and subjected to an in vitro kinase assay using a FAK-GST fusion protein as a substrate. For the immunofluorescence studies, CAM tissue underlying the disks were frozen in OCT, a cryopreservative, sectioned at 4 µm, fixed in acetone for 1 minute, incubated in 3% normal goat serum for 1 hour, followed by an incubation in primary rabbit anti-phosphorylated ERK antibody as described previously (Eliceiri et al., J. Cell Biol., 140:1255-1263 (1998), washed in PBS and detected with a fluorescent secondary antibody.

### A. Activation of Endogenous Src by bFGF or VEGF

To assess the effects of growth factors on Src activity in modulating angiogenesis, the following assays were performed. Tissue extracts of 10 day old chick CAMs that had been exposed to bFGF or VEGF (2 µg/ml) for 2 hours were lysed. Endogenous Src was immunoprecipitated from equivalent amounts of total protein and subjected to an in vitro immune complex kinase assay using a FAK-GST fusion protein as a substrate, electrophoresed and transferred to nitrocellulose.

The results of the assay are shown in FIG. 5 where the increase in Src activity is evident in the increased density of the gel with either bFGF or VEGF treatment as compared to untreated (mock) samples that are indicative of baseline Src activity in the CAM assay. Both bFGF and VEGF resulted in approximately a 2 fold increase of endogenous Src activity present in the CAM. The above kinase assay blot was also probed with an anti-Src antibody as a loading control for equivalent Src and IgG content.

### B. Effect of Retrovirus-Mediated Gene Expression of Src A on Angiogenesis in the Chick CAM

The following assay was performed to assess the effect of mutated Src proteins on angiogenesis in the CAM preparation. For the assay, 9 day old chick CAMs were exposed to RCAS-Src A or RCAS-GFP expressing retroviruses or buffer for 72 hour following the protocol described above.

The results of this assay are shown in FIG. 6A where the level of angiogenesis was quantified as described above. Representative photomicrographs (4x) were taken with a stereomicroscope as shown in FIG. 6B. Baseline endogenous Src activity has an angiogenic index of approximately 50. In contrast, CAMs treated with retroviral vector-expressed RCAS-Src A having a point mutation at amino acid residue position 527 from a tyrosine to a phenylalanine resulted in an enhancement (induction) of angiogenesis of an angiogenic index of approximately 90. The enhancement of Src-A mediated angiogenesis is also evident in the photographs shown in FIG. 6B.

### C. Retroviral Expression of Src A Activates Vascular MAP Kinase Phosphorylation

The effect of Src A as compared to growth factors VEGF and PMA on vascular MAP kinase phosphorylation was also assessed following the assay procedures described above and herein. Tissue extracts of 10 day old chick CAMs exposed to VEGF or PMA (another mitogen at a comparable concentration) for 30 minutes were compared to those infected with Src A-expressing retrovirus for 48 hours. Src was than immunoprecipitated from equivalent amounts of total protein extract and subjected to an in vitro immune complex kinase assay using a FAK-GST fusion protein as a substrate, electrophoresed and transferred to nitrocellulose.

The results of this assay are shown in FIG. 7A where untreated CAMs (NT) exhibit base-line endogenous Src-mediated vascular MAP kinase phosphorylation. Both VEGF and PMA resulted in an approximate 2 fold increase over baseline. In contrast, Src A enhanced the activity approximately 5 to 10 fold over that seen with untreated samples.

Aliquots of the above whole tissue lysates were also measured for endogenous ERK phosphorylation by immunoblotting with an anti-phospho-ERK antibody as shown in FIG. 7B. For this assessment, 10 day old CAMs were infected with either mock RCAS or RCAS that expresses SRC A. After two days, CAMs were dissected, cryopreserved in OCT and sectioned at 4 µm. Sections were immunostained with an anti-phosphorylated ERK antibody (New England Biolabs), washed and detected with a goat anti-rabbit FITC-conjugated secondary antibody. Fluorescent images were captured on a cooled-CCD camera (Princeton Inst.). The photomicrographs indicate enhanced immunofluorescence with Src A-treated preparations compared to mock controls.

### D. Selective Requirement for Src Activity During VEGF, but Not bFGF-Induced Angiogenesis

To assess the effect of Src modulatory activity on growth factor induced angiogenesis, the following assays were performed. Nine day old chick CAMs were exposed to the retroviral vector preparation that expressed the dominant negative Src mutation referred to as Src 251 or Src K295M as previously described. RCAS-Src 251 or control RCAS-GFP retroviruses or buffer CAMS were treated for 20 hours and then incubated for an additional 72 hours in the presence or absence of bFGF or VEGF.

The level of angiogenesis, quantified as described above, is shown in FIG. 8A. Representative photomicrographs (6x), shown in FIG. 8B, were taken with a stereomicroscope. FIG. 8C illustrates a blot probed with an anti-Src antibody to confirm the expression of Src 251 in transfected cells as compared to mock treatments.

The results of the assays described above indicate that both bFGF and VEGF treated CAMS in the presence of RCAS-GFP controls induced angiogenesis over the Src-mediated baseline angiogenesis seen with mock or untreated CAM preparations. The expressed dominant negative mutant Src 251 was effective at inhibiting VEGF-induced angiogenesis back to baseline levels while not effective at inhibiting bFGF-mediated angiogenesis. The photomicrographs shown in FIG. 8B pictorially confirm the data shown in FIG. 8A. Thus, retrovirally expressed Src 251 is an effective angiogenesis inhibitor, when angiogenesis is induced with VEGF.

Applications of the Src proteins useful in this invention with other angiogenesis models as described in the Examples below are contemplated in the present invention.

### 5. Repression of Tumor Tissue Growth With Src Modulators as Measured by In Vivo Rabbit Eye Model Assay

The effect of Src modulators on growth factor-induced angiogenesis can be observed in naturally transparent structures as exemplified by the cornea of the eye. New blood vessels grow from the rim of the cornea, which has a rich blood supply, toward the center of the cornea, which normally does not have a blood supply. Stimulators of angiogenesis, such as bFGF, when applied to the cornea induce the growth of new blood vessels from the rim of the cornea. Antagonists of angiogenesis, applied to the cornea, inhibit the growth of new blood vessels from the rim of the cornea. Thus, the cornea undergoes angiogenesis through an invasion of endothelial cells from the rim of the cornea into the tough collagen-packed corneal tissue which is easily visible. The rabbit eye model assay therefore provides an in vivo model for the direct observation of stimulation and inhibition of angiogenesis following the implantation of compounds directly into the cornea of the eye.

### In Vivo Rabbit Eye Model Assay Demonstrate Angiogenesis Induced by Growth Factors

Angiogenesis is induced in the in vivo rabbit eye model assay with growth factors bFGF or VEGF and is described in the following sections.

Hydron polymer pellets containing growth factor are prepared as described by D'Amato, et al., Proc. Natl. Acad. Sci., USA, 91:4082-4085 (1994). The individual pellets contain 650 ng of the growth factors separately bound to sucralfate (Carafet, Marion Merrell Dow Corporation) to stabilize the growth factor and ensure its slow release into the surrounding tissue. In addition, hydron pellets are prepared containing a desired Src-expressing retrovirus as previously described. The pellets are cast in specially prepared Teflon pegs that have a 2.5 mm core drilled into their surfaces. Approximately 12 µl of casting material is placed into each peg and polymerized overnight in a sterile hood. Pellets are then sterilized by ultraviolet irradiation. Effects of Src proteins are then assessed as previously described.

### 6. In Vivo Regression of Tumor Tissue Growth With Src Modulators As Measured by Chimeric Mouse:Human Assay

An in vivo chimeric mouse:human model is generated by replacing a portion of skin from a SCID mouse with human neonatal foreskin. The in vivo chimeric mouse:human model is prepared essentially as described in Yan, et al., J. Clin. Invest., 91:986-996 (1993). Briefly, a 2 cm² square area of skin is surgically removed from a SCID mouse (6-8 weeks of age) and replaced with a human foreskin. The mouse is anesthetized and the hair removed from a 5 cm² area on each side of the lateral abdominal region by shaving. Two circular graft beds of 2 cm² are prepared by removing the full thickness of skin down to the fascia. Full thickness human skin grafts of the same size derived from human neonatal foreskin are placed onto the wound beds and sutured into place. The graft is covered with a Band-Aid which is sutured to the skin. Micropore cloth tape is also applied to cover the wound.

The M21-L human melanoma cell line or MDA 23.1 breast carcinoma cell line (ATCC HTB 26; αᵥβ₃ negative by immunoreactivity of tissue sections with mAb LM609), are used to form the solid human tumors on the human skin grafts on the SCID mice. A single cell suspension of 5 x 10⁶ M21-L or MDA 23.1 cells is injected intradermally into the human skin graft. The mice are then observed for 2 to 4 weeks to allow growth of measurable human tumors.

After a measurable tumor is established, retrovirus preparations or PBS is injected into the mouse tail vein. Following a 2-3 week period, the tumor is excised and analyzed by weight and histology. The effect of expressed Src proteins on the tumors is then assessed.

### 7. In Vitro Regression of Human Tumor Tissue Growth With Src Modulators As Measured by CAM Assay

Tumor growth depends on angiogenesis (Folkman, 1992, J. Biol. chem. 267:10931-10934; Weidner et al., 1991, N.E. J. Med. 324:1-8; Brooks et al., 1994, Cell 79:1157-1164). In fact, recent reports suggest that tumor growth is susceptible to the anti-angiogenic effects of VEGF receptor antagonists (Kim et al., 1993, Nature 362:8451-844). Therefore, we examined whether suppression of angiogenesis by delivery of kinase-deleted Src 251 would influence the growth of a human medulloblastoma (DAOY), a highly angiogenic tumor known to produce VEGF and very little bFGF.

The 3 and 6 day DAOY medulloblastoma tumor growth assays were performed in the chick CAM essentially as previously described (Brooks et al., 1994, supra). 5 x 10⁶ DAOY cells cultured in RPMI 1640 containing 10% fetal calf serum were washed and seeded on the CAM of a 10 day embryo to produce DAOY tumor fragments. After 7 days 50 mg tumor fragments were dissected and reseeded on another 10 day embryo and incubated for another 3 or 6 days with the topical application (25µl) of either control RCAS-GFP retrovirus, RCAS-Src 251, or mock treatment. Using the whole tissue confocal imaging of infected tumors as a guide we were able to determine that there was significant expression of the RCAS constructs around and within the tumor fragment with this topical approach. Tumor resections and weighing were performed in a double blind manner removing only the easily definable solid tumor mass (Brooks et al., 1994, supra). The wet tumor weights after 3 or 6 days were compared with initial weight and the percent change of tumor weight determined for each group.

These tumors readily grow on the CAM and produces active angiogenesis (FIG. 9) allowing us to selectively target the avian-derived tumor vasculature by using an avian-specific RCAS retrovirus.

FIG. 9, depicts results that show retroviral delivery of RCAS-Src 251 to human tumors growing on the chick CAM reverses tumor growth. FIG. 9A shows human medulloblastomas that were grown on the CAM of chick embryos as described above. Retrovirus containing RCAS-GFP or RCAS-Src 251 was topically applied to preestablished tumors of greater than 50 mg. A representative micrograph of a medulloblastoma tumor fragment infected with RCAS-GFP expressing GFP reveals exclusive expression in the tumor blood vessels (arrowhead) as detected by optical sectioning with a Bio Rad laser confocal scanning microscope (bar=500µm). FIG. 9B shows results from tumors treated as above that were allowed to grow for 3 or 6 days after which they were resected and wet weights determined. Data are expressed as the mean change in tumor weight (from the 50 mg tumor starting weight) +/- SEM of 2 replicates. RCAS-Src 251 had a significant impact on tumor growth after 3 days (*, P<0.002) and 6 days (**, P<0.05). FIG. 9C shows representative stereomicrographs of medulloblastoma tumors surgically removed from the embryos which were taken with an Olympus stereomicroscope (bar=350µm). (Lower panel) A high magnification micrograph of each tumor showing the vasculature of each tumor in detail (bar=350µm). The arrowhead indicates blood vessel disruption in RCAS-Src251-treated tumors.

The results show that delivery of RCAS containing Src 251 to preestablished medulloblastomas resulted in selective viral expression in the tumor-associated blood vessels (FIG. 9A) and this ultimately led to the regression of these tumors within the span of six days (FIG. 9B). Importantly, the tumor-associated blood vessels in animals treated with virus containing Src 251 were severely disrupted and fewer in number compared to the tumor vessels in control animals (FIG. 9C). The fact that RCAS-GFP infected tumors showed GFP localization only in the tumor vasculature suggests that the anti-tumor effects observed with retrovirally delivered Src 251 were due to its anti-angiogenic properties.

### 8. Src Requirement for Endothelial Cell Survival during VGEF-, but not bFGF- Mediated Angiogenesis

Recent evidence suggests that growth factor receptors (Choi and Ballermann, 1995, J. Biol. Chem. 270:21144-21150; Satake et al., 1998, Biochem. Biophys. Res. Comm. 244:642-646) and integrins (Meredith et al., 1993, Mol.Biol.Cell 4:953-961; Brooks et al., 1994a, Science 264:569-571) promote survival of angiogenic endothelial cells. The fact that both growth factors and adhesion receptors also regulate Src activity prompted the examination of the role of Src in endothelial cell survival during angiogenesis. CAMs stimulated with either bFGF or VEGF were infected with retrovirus containing Src 251, and cryostat sections of these tissues were examined for the presence of apoptotic cells.

Briefly, cryosections of CAMs treated with RCAS-GFP or RCAS-Src 251 treated with bFGF or VEGF were analyzed for apoptic cells using the Apoptag Kit (Oncor, Gaithersburg, MD). Sections were also immunostained with a rabbit polyclonal anti-vWf (biogenix, San Ramon, CA) and counterstained with 1 ug/ml DAPI. Fluorescent images were captured with a cooled CCD camera (Roper, Trenton, NJ), and the fluorescent images were processed and exposure matched between experimental treatments as previously described (Ellcelri et al. 1998, supra).

To measure the apoptic index of retrovirus-infected CAM tissues, FITC-conjugated annexin V (Clontech, Palo Alto, CA) was used to stain cell suspensions, and the washed cells were analyzed by flow cytometry. Cell suspensions of CAM cells were prepared from mock- or virus-infected CAMs by digestion with 0.1% (w/v) collagenase type IV (Worthington Biochemicals, Lakewood, NJ), in RPMI 1640 of minced CAM tissue rocking for 1 hr at 37°C as previously described (Brooks et al., 1994b) and filtered through 100 µm nylon mesh (Becton Dickinson, Fountain Lakes, NJ). Fluorescence was measured with a FACscan flow cytometer (Becton Dickinson) to count 10,000 cells.

Measurement of vWf staining by FACs was performed with parallel collagenase digested CAM tissue cell preparations, that were fixed in 1.8% paraformaldehyde, permeabilized in 70% ethanol, incubated the anti-vWf antibody, and detected with a FITC-conjugated secondary antibody.

Delivery of Src 251 promoted extensive TUNEL staining among the factor VIII-related antigen (von Willebrand factor [vWf]) positive blood vessels in VEGF-but not bFGF-, stimulated CAMS. In fact, minimal apoptosis was observed among other cell types in these CAMs, suggesting an endothelial cell-specific requirement for Src kinase activity for cell survival in VEGF-activated blood vessels. In a second series of experiments, retrovirus-infected CAMs stimulated with VEGF or bFGF were subjected to limited collagenase digestion to prepare a single cell suspension. These CAM-derived cells were shown to contain approximately 20%-50% endothelial cells (vWf positive) and analyzed for apoptosis by flow cytometric detection of annexin V-positive cells, an early apoptosis marker. Cells derived from VEGF-stimulated CAMs infected with Src 251 had significantly increased annexin V staining relative to cells from mock RCAS-GFP-infected CAMs treated with VEGF. In contrast, cells derived from mock-infected CAMs or those infected with RCAS-Src 251 and stimulated with bFGF exhibited little or no annexin V staining. In addition, no annexin V staining was detected among cells derived from nonstimulated or bFGF-stimulated CAMs. These data demonstrate that Src kinase activity is selectively required for endothelial cell survival during VEGF, but not bFGF-mediated angiogenesis in the CAM.

### 9. Selective Requirement for Src Kinase Activity In an Subcutaneous Murine Model of Angiogenesis

To further analyze the role of Src in angiogenesis, a murine model was employed. In this case, angiogenesis was induced by subcutaneous injection of growth factor-depleted Matrigel supplemented with either bFGF (100 ng/ml) or VEGF (400 ng/ml) in athymic wehl(nu/nu) adult mice and analyzed after 5 days (Passaniti et al., 1992). Angiogenesis was quantitated by removing and homogenizing tissue, isolating the proteins, and immunoblotting with a VEGF receptor antibody (flk-1) (FIG. 13A) that is specific for endothelial cells. As observed in the chick, expression of the kinase-deleted Src 251 cDNA blocked VEGF-induced angiogenesis in this murine model while having no effect on bFGF-induced angiogenesis (FIG. 13B). To establish the role of endogenous Src in this model, tissues were infected with a retrovirus expressing Cak that inhibits endogenous Src activity by phosphorylation of the C-terminal regulatory site (Nada et al., 1991, Nature 361:68-72). Expression of Cak blocked VEGF-, but not bFGF-, induced angiogenesis (FIG. 13), confirming a role for endogenous Src activity in VEGF-mediated angiogenesis. Neovascularization of these virus-infected VEGF-stimulated tissues was confirmed by direct immunofluorescence with a FITC-conjugated anti-DC34 antibody (FIG. 13) or an anti-flk-1 antibody and quantitated by enumerating the number of positively stained CD34 blood vessels in each cryosection (FIG. 13C).

Briefly, angiogenesis was induced by a subcutaneous injection of growth factor depleted Matrigel containing saline or VEGF (400 ng/ml) with 2x 10⁶ ectropic packaging cells expressing GFP retrovirus in the flank of athymic wahl (nu/nu) mice and analyzed after 5 days of incubation. The neovascularization was quantitated by immunoblotting with a VEGF receptor antibody (flk-1) that is specific for endothelial cells. FIG. 15A depicts immunoblotting results. The effects of kinase-deleted Src-251, Csk, or GFP retrovirus on VEGF- (400 ng/ml) or bFGF- (400 ng/ml) induced angiogenesis was analyzed by immunoblotting the tissue lysates with an anti flk-1 antibody. An example of these results are depicted in FIG. 15B. The effect of the Src 251- and Csk-expressing retroviruses on VEGF-induced neovascularization was quantified by enumerating the number of CD34 positive vessels in tissue cross sections by indirect immunofluorescence in triplicate random fields at 20x. Cryosections of the plugs were also subjected to immunofluorescent staining with an anti-CD34 antibody or an anti-flk antibody, photographed, and quantitated as described above for the CAM angiogenesis assays.

Whole-mount direct fluorescence of RCAS-GFP-infected tumor fragment was accomplished by dissecting a tumor fragment and imaging the unfixed tissue directly on a slide with a laser confocal microscope (MRC 1024: Bio-Rad, Hercules, CA).

### 10. The Effect of Intradermal Expression of VEGF In src^{-/-} or src^{+/-} Mice Ears

Continuing the results obtained with chicken and mouse angiogenesis models, a direct genetic approach was employed to examine intradermal VEGF-induced angiogenesis in src^{-/-} mice. Also examined were effects on vascular permeability, since it was known that VEGF both initiates new blood vessel growth and can promote vascular permeability (Senger et al., 1983 Science 218:983-985; Ferrera and Davis-Smyth, 1997, Endocr. Rev. 16:4-25).

Intradermal injections of adenovirus expressing a human VEGF cDNA were performed in the ear of src^{+/-} and src^{+/-} mice, while control β-galactosidase expressing adenovirus was injected into the opposite ear of each mouse. VEGF-dependent new blood vessel growth in src^{+/-} ears was first detectable within 48 hr, and neovascularization was analyzed after 5 days.

Briefly, pp60^{c-src}, pp60^{c-yes}, pp60^{c-fyn}, deficient mice (129/8v/Ev x C57B16/J) were generated as previously described (Soriano et al., 1991, Cell 64:693-702). Additional stocks were obtained from Jackson labs. Mouse ears were injected intradermally (Eriksson et al., 1980, Microvasc. Res. 19:374-378) with 5 µl of adenovirus expressing either VEGF or β-galactosidase and the ears photographed after 5 days, with a stereoscope.

It was found that there were identical viral expression levels in src^{+/-} and src^{-/-} as determined by X-gal staining of β-galactosidase-adenovirus injected ears. In VEGF-injected src^{-/-} ears, there was no significant decrease in angiogenesis as measured by counting branch points (p<0.05). However, surprisingly, the most apparent phenotype in these animals was the complete blockade of vascular leakage compared to the VEGF-injected src^{+/-} ears. Examination of ears injected with VEGF confirms the extent of the vascular leakage in src^{+/-} mice, that is essentially absent in the src^{-/-} mice. The vascular leakage in these animals suggested that the VP activity, which has been associated with angiogenesis in vivo (Dvorak et al., 1995, Am. J. Pathol. 148:1029-1039), could be selectively disrupted in pp60^{c-src} deficient mice.

### 11. VEGF Fails to Compromise the Blood-Brain Barrier in Mice Lacking pp60^{c-sro}

The brain vasculature is characterized by a highly restrictive blood-brain barrier that prohibits small molecules from extravasating into the surrounding brain tissue. Tumor growth within the brain can compromise this barrier due in part to the production of angiogenic growth factors such as VEGF. Therefore, we examined the nature of the blood-brain barrier in src^{+/-} or src^{-/-} mice. In this case, VEGF or saline was stereotactically injected into the right or left hemisphere of the brain, respectively. All mice received systemic injections of Evan's blue dye to monitor VP activity.

Briefly, saline or VEGF (200 ng in 2 ul) was injected stereotactically into the left or right frontal lobe 92 mm to the left/right of the midline, 0.5 mm rostral from bregma, and 3 mm in depth from the dura, respectively. The animals received an Evan's blue dye solution intravenously 30 min after injection, as described above. After an additional 30 min, the mice were perfused and the brains were removed. Evan's blue dye fluorescence was observed using confocal laser microscopy of fresh unfixed cryosections of the brain.

Vascular leakage of blood was localized to the VGEF-injected hemisphere in src^{+/-} mice, but there was a complete absence of vascular leakage in src^{-/-} mice. This was also the case when examing the VP by measuring the accumulation of Evan's blue dye as detected by epifluoresence analysis of cryostat sections of these brains. Thus, VEGF compromises the blood-brain barrier in a manner that depends on active pp60^{c-sro}.

### 12. VEGF-Mediated VP, but Not Inflammation-Associated VP, Depends on pp60^{c-sro}

To further analyze and quantitate the effect of VEGF as a VP factor in src^{+/-} or src^{-/-} mice, a Miles assay (Miles & Miles, 1952) was used to quantitatively measure the vascular permeability in the skin of these animals. VEGF was injected intradermally in src^{+/-} or src^{-/-} mice that had received an intravenous systemic administration of Evan's blue dye. Within 15 min after injection of VEGF, there was a 3-fold increase in VP in src^{+/-} mice. However, in src^{-/-} mice no detectable VP activity was observed. Dye elution of the injected skin patches were quantitated and compared with control saline and bFGF. bFGF or saline controls injected adjacent to the VEGF showed no significant increase in VP.

Briefly, the Miles assay (Miles et al., 1962) was adapted for mice by injecting 10 µl of VEGF (400 ng/ml), allyl isothiocyanate (mustard oil, 20% w/v in mineral oil), or saline intradermally into mice that had previously been intravenously injected with 100 µl of 0.5% Evan's blue dye. After 15 min, the skin patches were dissected, photographed, and eluted at 58°C with formalin and quantitated with a spectrophotometer.

Vascular leakage/permeability is also known to occur during inflammation, which allows for the accumulation of serum-associated adhesive protein and inflammatory cells in tissues. In fact, inflammatory mediators themselves directly promote vascular leakage. Therefore, one such inflammatory mediator, allyl isothiocyanate, also known as mustard oil (Inoue et al., 1997, supra), was tested in src^{+/-} or src^{-/-} mice for its capacity to produce VP. Unlike that observed in VEGF-stimulated src^{-/-} animals, no decrease in the VP produced by the injection of the inflammatory mediator allyl isothiocyanate was detected. Thus, it can be concluded that Src plays a selective role in the VP activity induced with VEGF and does not influence VP associated with the inflammatory process.

### 13. VEGF-Mediated VP Activity Depends on Src and Yes, but not Fyn

The specificity of the Src requirement for VP was explored by examining the VEGF-induced VP activity associated with SFKs such as Fyn or Yes, which, like Src, are known to be expressed in endothelial cells (Bull et al., 1994, FEBS Letters, 361:41-44; Kiefer et al., 1994, Curr. Biol. 4:100-109). It was confirmed that these three SFKs were expressed equivalently in the aortas of wild-type mice. Like src^{-/-} mice, animals deficient in Yes were also defective in VEGF-induced VP. However, surprisingly, mice lacking Fyn retained a high VP in response to VEGF that was not significantly different from control animals. The disruption of VEGF-induced VP in src^{-/-} or yes^{-/-} mice demonstrates that the kinase activity of specific SFKs is essential for VEGF-mediated signalling event leading to VP activity but not angiogenesis.

The vascular permeability properties of VEGF in the skin of src^{+/-} (FIG. 14A, left panel) or src^{-/-} (FIG. 14A, right panel) mice was determined by intradermal injection of saline or VEGF (400 ng) into mice that have been intravenously injected with Evan's blue dye. After 15 min, skin patches were photographed (scale bar, 1 mm). The stars indicate the injection sites. The regions surrounding the injection sites of VEGF, bFGF or saline were dissected, and the VP quantitated by elution of the Evan's blue dye in formamide at 58°C for 24 hr, and the absorbance measured at 500 nm (FIG. 14B, left graph). The ability of an inflammation mediator (allyl isothiocyanate), known to induce inflammation related VP, was tested in src^{+/-} or src^{-/-} mice (FIG. 14B, right).

The ability of VEGF to induce VP was compared in src^{-/-}, fyn^{-/-}, or yes^{-/-} mice in the Miles assay (FIG. 14C). Data for each of the Miles assays are expressed as the mean ± SD of triplicate animals. src^{-/-} and yes^{-/-} VP defects compared to control animals were statistically significant (*p <0.05, paired t test), whereas the VP defects in neither the VEGF-treated fyn^{-/-} mice nor the allyl isothiocyanate treated src^{+/-} mice were statistically significant (**p<0.05).

### 14. Src family tyrosine kinase inhibitor treated mice, and Src -/- mice show reduced tissue damage associated with trauma or injury to blood vessels than untreated wild-type mice

Specific administration of inhibitors of the Src family kinases acts as inhibitors of pathological vascular leakage and permeability during vascular injury or disorders such as stroke. The vascular endothelium is a dynamic cell type that responds to many cues to regulate processes such as the sprouting of new blood vessels during angiogenesis of a tumor, to the regulation of the permeability of the vessel wall during stroke-induced edema and tissue damage.

Reduction of vascular permeability in two mouse stroke models, by drug inhibition of the Src pathway, is sufficient to inhibit brain damage by reducing ischemia-induced vascular leak. Furthermore, in mice genetically deficient in Src, which have reduced vascular leakage/permeability, infarct volume is also reduced. The combination of the synthetic Src inhibitor data, with the supporting genetic evidence of reduced the vascular leakage in stroke and other related models demonstrates the physiological relevance of this approach in reducing brain damage following strokes. Inhibition of these pathways with a range of available Src family kinase inhibitors of these signaling cascades has the therapeutic benefit of mitigating brain damage from vascular permeability-related tissue damage.

Two different methods for induction of focal cerebral ischemia were used. Both animal models of focal cerebral ischemia are well established and widely used in stroke research. Both models have been previously used to investigate the pathophysiology of cerebral ischemia as well as to test novel antistroke drugs.
a) Mice were anesthetized with avertin and body temperature was maintained by keeping the animal on a heating pad. An incision was made between the right ear and the right eye. The scull was exposed by retraction of the temporal muscle and a small burr hole was drilled in the region over the middle cerebral artery (MCA). The meninges were removed and the right MCA was occluded by coagulation using a heating filament. The animals were allowed to recover and were returned to their cages. After 24 hours, the brains were perfused, removed and cut into 1 mm cross-sections. The sections were immersed in 2% 2,3,5-triphenyltetrazolium chloride (TTC) and the infarcted brain area was identified as unstained (white) tissue surrounded by viable (red) tissue. The infarct volume was defined as the sum of the unstained areas of the sections multiplied by their thickness.
   Mice deficient in Src (Src-/-) were used to study the role of Src in cerebral ischemia. Src+/- mice served as controls. We found that in Src-/- mice the infarct volume was reduced from 26 ± 10 mm³ to 16 ± 4 mm³ in controls 24 hours after the insult. The effect was even more pronounced when C57B16 wild-type mice were injected with 1.5 mg/kg PP1 intraperitoneally (i.p.) 30 min after the vessel occlusion. The infarct size was reduced from 31 ± 12 mm³ in the untreated group to 8 ± 2 mm³ in the PP1-treated group.
b) In a second model of focal cerebral ischemia the MCA was occluded by placement of an embolus at the origin of the MCA. A single intact fibrin-rich 24 h old homologous clot was placed at the origin of the MCA using a modified PE-50 catheter. Induction of cerebral ischemia was proven by the reduction of cerebral blood flow in the ipsilateral hemisphere compared to the contralateral hemisphere. After 24 hours the brains were removed, serial sections were prepared and stained with hematoxylin-eosin (HE). Infarct volumes were determined by adding the infarct areas in serial HE sections multiplied by the distance between each section.

The dosage of PP1 used in this study (1.5 mg/kg i.p.) was empirically chosen. It is known that VEGF is first expressed about 3 hours after cerebral ischemia in the brain with a maximum after 12 to 24 hours. In this study PP1 was given 30 min after the onset of the infarct to completely block VEGF-induced vascular permeability increase. According to the time course of typical VEGF expression, a potential therapeutical window for the administration of Src-inhibitors would be up to 12 hours after the stroke. In diseases associated with a sustained increase in vascular permeability a chronic administration of the Src inhibiting drug is appropriate.

FIG. 15 is a graph which depicts the comparative results of averaged infarct volume (mm³) in mouse brains after injury, where mice were heterogeneous Src (Src +/-), dominant negative Src mutants (Src -/-), wild type mice (WT), or wild type mice treated with 1.5 mg/kg PP1 (PP1).

FIG. 16 illustrates sample sequential MRI scans of isolated perfused mouse brain after treatment to induce CNS injury, where the progression of scans in the PP1 treated animal (right) clearly shows less infarct than the progression of scans in the control untreated animal (left).

The present invention is particularly suited for the specific intervention of VP induced tissue damage because the targeted inhibition of Src family tyrosine kinase action focuses inhibition on VP without long term effect on other VEGF-induced responses which can be beneficial to recovery from injury. In contrast to neutralizing VEGF protein, the inhibition of Src does not interfere with the cumulative angiogenic effect of VEGF which might be beneficial in a later stage of the disease.

The use of synthetic small-molecule inhibitors is in general safer and more manageable that the use of large proteins. The use of recombinant proteins, such as a VEGF receptor-murine Immunoglobulin fusion protein is potentially harmful, and does not allow for repeated administration for fear of provoking an allergic reaction when used in humans (i.e.. Human anti-mouse antibody; HAMA).

Finally, VEGF is not the only activator of downstream Src, other cytokines involved in the pathophysiology of cerebral ischemia which can influence vascular permeability, such as IL-6 and TNF-α. Thus, inhibition of VEGF may not inhibit all subsequent injury related Src activation. In fact, reduction of infarct size by PP1 is more pronounced than by VEGF antagonism indicating that other pathways may activate Src kinases facilitating permeability increase.

The foregoing written specification is considered to be sufficient to enable one skilled in the art to practice the invention. The deposit of material does not constitute an admission that the written description herein contained is inadequate to enable the practice of any aspect of the invention, including the best mode thereof, nor is it to be construed as limiting the scope of the claims to the specific illustration that it represents. Indeed, various modifications of the invention in addition to those shown and described herein will become apparent to those skilled in the art from the foregoing description and fall within the scope of the appended claims.

## Claims

1. A pharmaceutical composition comprising tyrosine kinase proteins Src and Yes, together with a pharmaceutically acceptable carrier.

2. The pharmaceutical composition of claim 1 wherein said Src protein is Src-A, or has at amino acid residue 527 any amino acid residue except for tyrosine, serine or threonine, or wherein said Src protein is inactive.

3. The pharmaceutical composition of claim 2 wherein said inactive Src protein is Src K295M, or Src 251.

4. The pharmaceutical composition of claim 1 wherein said Yes protein is an inactive Yes protein.

5. A pharmaceutical composition comprising an inactive tyrosine kinase protein Yes or a mixture of inactive tyrosine kinase proteins Src and Yes for use in a method for modulating vascular permeability in a tissue suffering from a disease condition and comprising contacting said tissue with a therapeutically effective VP modulating amount of said pharmaceutical composition, wherein the disease condition is selected from the group consisting of stroke induced damages, growth of tumors, myocardial infarction, arteriosclerosis, brain edema, cerebrovascular disease or trauma, rheumatoid arthritis, diabetic retinopathy, arthritis, inflammatory diseases, chronic articular rheumatism and psoriasis, restenosis, cerebral hemorrhage, brain and spinal trauma, hypoxia-induced brain and spinal injury, inflammatory disorders of the CNS, viral or bacterial infections of the CNS, autoimmune disorders, diseases with a chronic increase in blood brain barrier permeability, adult respiratory distress syndrome (ARDS), and metastasis formation.

6. A pharmaceutical composition comprising an active tyrosine kinase protein Yes or a mixture of active tyrosine kinase proteins Src and Yes for use in a method for modulating vascular permeability in a tissue suffering from a disease condition and comprising contacting said tissue with a therapeutically effective VP modulating amount of said pharmaceutical composition, wherein the disease condition is selected from the group consisting of ischemic limbs and chronic wounds.

7. An article of manufacture comprising packaging material and a pharmaceutical composition contained within said packaging material, wherein said pharmaceutical composition is capable of modulating vascular permeability in a tissue suffering from a disease condition, wherein said packaging material comprises a label which indicates that said pharmaceutical composition can be used for treating disease conditions by modulating vascular permeability, and wherein said pharmaceutical composition comprises a tyrosine kinase Src protein and Yes protein, in a pharmaceutically acceptable carrier.

8. An article of manufacture comprising packaging material and a pharmaceutical composition contained within said packaging material, wherein said pharmaceutical composition is capable of modulating vascular permeability in a tissue suffering from a disease condition, wherein said packaging material comprises a label which indicates that said pharmaceutical composition can be used for treating disease conditions by modulating vascular permeability, and wherein said pharmaceutical composition comprises a tyrosine kinase Yes protein, in a pharmaceutically acceptable carrier.

9. A pharmaceutical composition comprising a Src family tyrosine kinase inhibitor for use in a method for ameliorating tissue damage related to vascular leakage or edema comprising contacting said tissue with a vascular permeability modulating amount of said pharmaceutical composition, wherein the Src family tyrosine kinase inhibitor is selected from the group consisting of an inactive Yes protein, a mixture of an inactive Src protein and an inactive Yes protein, and a mixture of an inactive Yes protein and/or an inactive Src protein together with a chemical inhibitor selected from PP1, PP2, PD173955, AGL1872, PD162531, Radicicol R2146, and Geldanamycin.

10. An article of manufacture comprising packaging material and a pharmaceutical composition contained within said packaging material, wherein said pharmaceutical composition is capable of modulating vascular permeability increase in a tissue suffering from a disease condition, wherein said packaging material comprises a label which indicates that said pharmaceutical composition can be used for treatment of vascular leakage or edema associated disease conditions, and wherein said pharmaceutical composition comprises a Src family tyrosine kinase inhibitor and a pharmaceutically acceptable carrier therefore, wherein the Src family tyrosine kinase inhibitor is selected from the group consisting of an inactive Yes protein, a mixture of an inactive Src protein and an inactive Yes protein, and a mixture of an inactive Yes protein and/or an inactive Src protein together with a chemical inhibitor selected from PP1, PP2, PD173955, AGL1872, PD162531, Radicicol R2146, and Geldanamycin.

## Patentansprüche

1. Pharmazeutische Zusammensetzung, die Tyrosinkinase-Proteine Src und Yes zusammen mit einem pharmazeutisch verträglichen Träger umfasst.

2. Pharmazeutische Zusammensetzung nach Anspruch 1, wobei das Src-Protein Src-A ist oder an dem Aminosäurerest 527 einen jeglichen Aminosäurerest mit der Ausnahme von Tyrosin, Serin oder Threonin aufweist oder wobei das Src-Protein inaktiv ist.

3. Pharmazeutische Zusammensetzung nach Anspruch 2, wobei das inaktive Src-Protein Src K295M oder Src 251 ist.

4. Pharmazeutische Zusammensetzung nach Anspruch 1, wobei das Yes-Protein ein inaktives Yes-Protein ist.

5. Pharmazeutische Zusammensetzung, die ein inaktives Tyrosinkinase-Protein Yes oder ein Gemisch von inaktiven Tyrosinkinase-Proteinen Src und Yes umfasst, zur Verwendung in einem Verfahren, das vaskuläre Permeabilität in einem Gewebe, das unter einem Erkrankungszustand leidet, moduliert und ein In-Kontakt-Bringen des Gewebes mit einer therapeutisch wirksamen VP-modulierenden Menge der pharmazeutischen Zusammensetzung umfasst, wobei der Erkrankungszustand aus der Gruppe ausgewählt ist, bestehend aus Schlaganfall-induzierten Schädigungen, Wachstum von Tumoren, Myokardinfarkt, Arteriosklerose, Gehirnödem, zerebrovaskulärer Erkrankung oder zerebrovaskulärem Trauma, rheumatoider Arthritis, diabetischer Retinopathie, Arthritis, entzündlichen Erkrankungen, chronischem Gelenkrheumatismus und Psoriasis, Restenose, Hirnblutung, Gehirn- und Wirbelsäulentrauma, Hypoxie-induzierter Gehirn- und Wirbelsäulenverletzung, entzündlichen Erkrankungen des ZNS, viralen oder bakteriellen Infektionen des ZNS, Autoimmunerkrankungen, Erkrankungen mit einer chronischen Zunahme der Permeabilität der Blut-Hirn-Schranke, akutem progressivem Lungenversagen (ARDS) und Metastasenbildung.

6. Pharmazeutische Zusammensetzung, die ein aktives Tyrosinkinase-Protein Yes oder ein Gemisch von aktiven Tyrosinkinase-Proteinen Src und Yes umfasst, zur Verwendung in einem Verfahren, das vaskuläre Permeabilität in einem Gewebe, das unter einem Erkrankungszustand leidet, moduliert und ein In-Kontakt-Bringen des Gewebes mit einer therapeutisch wirksamen VP-modulierenden Menge der pharmazeutischen Zusammensetzung umfasst, wobei der Erkrankungszustand aus der Gruppe ausgewählt ist, bestehend aus ischämischen Extremitäten und chronischen Wunden.

7. Herstellungsartikel, der Verpackungsmaterial und eine in dem Verpackungsmaterial enthaltene pharmazeutische Zusammensetzung umfasst, wobei die pharmazeutische Zusammensetzung zur Modulierung von vaskulärer Permeabilität in einem Gewebe, das an einem Erkrankungszustand leidet, fähig ist, wobei das Verpackungsmaterial einen Hinweis umfasst, der anzeigt, dass die pharmazeutische Zusammensetzung für eine Behandlung von Erkrankungszuständen durch Modulierung von vaskulärer Permeabilität verwendet werden kann, und wobei die pharmazeutische Zusammensetzung ein Tyrosinkinase-Src-Protein und -Yes-Protein in einem pharmazeutisch verträglichen Träger umfasst.

8. Herstellungsartikel, der Verpackungsmaterial und eine in dem Verpackungsmaterial enthaltene pharmazeutische Zusammensetzung umfasst, wobei die pharmazeutische Zusammensetzung zur Modulierung von vaskulärer Permeabilität in einem Gewebe, das unter einem Erkrankungszustand leidet, fähig ist, wobei das Verpackungsmaterial einen Hinweis umfasst, der anzeigt, dass die pharmazeutische Zusammensetzung für eine Behandlung von Erkrankungszuständen durch Modulierung von vaskulärer Permeabilität verwendet werden kann, und wobei die pharmazeutische Zusammensetzung ein Tyrosinkinase-Yes-Protein in einem pharmazeutisch verträglichen Träger umfasst.

9. Pharmazeutische Zusammensetzung, die einen Src-Familie-Tyrosinkinase-Inhibitor umfasst, zur Verwendung in einem Verfahren zum Verbessern eines Gewebeschadens, der mit einer Gefäßblutung oder einem Ödem in Beziehung steht, wobei das Verfahren ein In-Kontakt-Bringen des Gewebes mit einer die vaskuläre Permeabilität modulierenden Menge der pharmazeutischen Zusammensetzung umfasst, wobei der Src-Familie-Tyrosinkinase-Inhibitor aus der Gruppe ausgewählt ist, bestehend aus einem inaktiven Yes-Protein, einem Gemisch eines inaktiven Src-Proteins und eines inaktiven Yes-Proteins und einem Gemisch eines inaktiven Yes-Proteins und/oder eines inaktiven Src-Proteins zusammen mit einem chemischen Inhibitor, ausgewählt aus PP1, PP2, PD173955, AGL1872, PD 162531, Radicicol R2146 und Geldanamycin.

10. Herstellungsartikel, der Verpackungsmaterial und eine in dem Verpackungsmaterial enthaltene pharmazeutische Zusammensetzung umfasst, wobei die pharmazeutische Zusammensetzung zur Modulierung einer Zunahme von vaskulärer Permeabilität in einem Gewebe, das unter einem Erkrankungszustand leidet, fähig ist, wobei das Verpackungsmaterial einen Hinweis umfasst, der anzeigt, dass die pharmazeutische Zusammensetzung für eine Behandlung von mit einer Gefäßblutung oder einem Ödem in Beziehung stehenden Erkrankungszuständen verwendet werden kann, und wobei die pharmazeutische Zusammensetzung einen Src-Familie-Tyrosinkinase-Inhibitor und einen pharmazeutische verträglichen Träger dafür umfasst, wobei der Src-Familie-Tyrosinkinase-Inhibitor aus der Gruppe ausgewählt ist, bestehend aus einem inaktiven Yes-Protein, einem Gemisch eines inaktiven Src-Proteins und eines inaktiven Yes-Proteins und einem Gemisch eines inaktiven Yes-Proteins und/oder eines inaktiven Src-Proteins zusammen mit einem chemischen Inhibitor, ausgewählt aus PP1, PP2, PD173955, AGL1872, PD162531, Radicicol R2146 und Geldanamycin.

## Revendications

1. Composition pharmaceutique comprenant des protéines Src et Yes de thyrosine kinase, conjointement avec un support pharmaceutiquement acceptable.

2. Composition pharmaceutique selon la revendication 1, dans laquelle ladite protéine Src est Src-A, ou a sur le résidu d'acide aminé 527 un résidu d'acide aminé quelconque, à l'exception de la tyrosine, la sérine ou la thréonine, ou dans laquelle ladite protéine Src est inactive.

3. Composition pharmaceutique selon la revendication 2, dans laquelle ladite protéine Src inactive est Src K295M, ou Src 251.

4. Composition pharmaceutique selon la revendication 1, dans laquelle ladite protéine Yes est une protéine Yes inactive.

5. Composition pharmaceutique comprenant une protéine Yes de thyrosine kinase inactive, ou un mélange de protéines Src et Yes de thyrosine kinase inactives pour utilisation dans un procédé pour la modulation de la perméabilité vasculaire dans un tissu souffrant d'un état maladif et comprenant la mise en contact dudit tissu avec une quantité thérapeutiquement efficace pour moduler la VP de ladite composition pharmaceutique, tandis que l'état maladif est choisi dans le groupe consistant en les lésions provoquées par un ictus, la croissance de tumeurs, l'infarctus du myocarde, l'artériosclérose, l'oedème cérébral, les accidents ou traumatismes vasculaires cérébraux, la polyarthrite rhumatoïde, la rétinopathie diabétique, l'arthrite, les affections inflammatoires, le rhumatisme articulaire chronique et le psoriasis, la resténose, l'hémorragie cérébrale, les traumatismes cérébraux et médullaires, les lésions cérébrales et médulaires induites par une hypoxie, les troubles inflammatoires du SNC, les infections virales ou bactériennes du SNC, les troubles auto-immuns, les affections avec augmentation chronique de la perméabilité de la barrière hémato-encéphalique, le syndrome de détresse respiratoire de l'adulte (SDRA) et la formation de métastases.

6. Composition pharmaceutique comprenant une protéine Yes de tyrosine kinase active ou un mélange de protéines Yes et Src de tyrosine kinase actives, pour utilisation dans un procédé pour la modulation de la perméabilité vasculaire dans un tissu souffrant d'un état maladif et comprenant la mise en contact dudit tissu avec une quantité thérapeutiquement efficace de ladite composition pharmaceutique, modulant la VP, tandis que l'état maladif est choisi dans le groupe consistant en les membres ischémiques et les plaies chroniques.

7. Article manufacturé comprenant un matériau d'emballage et une composition pharmaceutique contenue dans ledit matériau d'emballage, dans lequel ladite composition pharmaceutique est capable de moduler la perméabilité vasculaire dans un tissu souffrant d'un état maladif, tandis que ledit matériau d'emballage comprend une étiquette qui indique que ladite composition pharmaceutique peut être utilisée pour le traitement d'états maladifs par modulation de la perméabilité vasculaire, et tandis que ladite composition pharmaceutique comprend une protéine Src et une protéine Yes de thyrosine kinase, dans un support pharmaceutiquement acceptable.

8. Article manufacturé comprenant un matériau d'emballage et une composition pharmaceutique contenue dans ledit matériau d'emballage, dans lequel ladite composition pharmaceutique est capable de moduler la perméabilité vasculaire dans un tissu souffrant d'un état maladif, tandis que ledit matériau d'emballage comprend une étiquette qui indique que ladite composition pharmaceutique peut être utilisée pour le traitement d'états maladifs par modulation de la perméabilité vasculaire, et tandis que ladite composition pharmaceutique comprend une protéine Yes de thyrosine kinase, dans un support pharmaceutiquement acceptable.

9. Composition pharmaceutique comprenant un inhibiteur de tyrosine kinase de la famille Src pour utilisation dans un procédé pour l'amélioration des atteintes aux tissus en relation avec une fuite vasculaire ou un oedème, comprenant la mise en contact dudit tissu avec une quantité modulant la perméabilité vasculaire de ladite composition pharmaceutique, tandis que l'inhibiteur de tyrosine kinase de la famille Src est choisi dans le groupe consistant en une protéine Yes inactive, un mélange d'une protéine Src inactive et d'une protéine Yes inactive, et un mélange d'une protéine Yes inactive et/ ou d'une protéine Src inactive, conjointement avec un inhibiteur chimique choisi parmi PP1, PP2, PD173955, AGL1872, PD162531, Radicicol R2146, et Geldanamycine.

10. Article manufacturé comprenant un matériau d'emballage et une composition pharmaceutique contenue dans ledit matériau d'emballage, dans lequel ladite composition pharmaceutique est capable de moduler l'augmentation de perméabilité vasculaire dans un tissu souffrant d'un état maladif, tandis que ledit matériau d'emballage comprend une étiquette qui indique que ladite composition pharmaceutique peut être utilisée pour le traitement d'états maladifs associés à une fuite vasculaire et à un oedème, et tandis que ladite composition pharmaceutique comprend un inhibiteur de thyrosine kinase de la famille Src et un support pharmaceutiquement acceptable pour celui-ci, tandis que l'inhibiteur de tyrosine kinase de la famille Src est choisi dans le groupe consistant en protéine Yes inactive, un mélange d'une protéine Src inactive et d'une protéine Yes inactive, et un mélange d'une protéine Yes inactive et/ou d'une protéine Src inactive conjointement avec un inhibiteur chimique choisi parmi PP1, PP2, PD173955, AGL1872, PD162531, Radicicol R2146, et Geldanamycine.
